# EUROPÄISCHE PATENTSCHRIFT

(11) **EP 0 873 313 B1**
(45) Veröffentlichungstag und Bekanntmachung des Hinweises auf die Patenterteilung: **15.03.2000**
(21) Anmeldenummer: 96934777.2
(22) Anmeldetag: 22.10.1996
(51) Int. Cl.: C07D 213/54, A01N 43/40

(54) **PYRIDYLESSIGSÄUREDERIVATE, VERFAHREN UND ZWISCHENPRODUKTE ZU IHRER HERSTELLUNG UND IHRE VERWENDUNG**
PYRIDYLACETIC ACID DERIVATIVES, PROCESS AND INTERMEDIATE PRODUCTS FOR THEIR PREPARATION, AND THEIR USE
DERIVES D'ACIDE PYRIDYLACETIQUE, PROCEDES ET PRODUITS INTERMEDIAIRES PERMETTANT DE LES PREPARER ET UTILISATION

(30) Priorität: 02.11.1995 DE 19540734
(43) Veröffentlichungstag der Anmeldung: 28.10.1998
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); BAYER, Herbert, D-68159 Mannheim (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); OBERDORF, Klaus, D-69117 Heidelberg (DE); RÖHL, Franz, D-67105 Schifferstadt (DE); GÖTZ, Norbert, D-67547 Worms (DE); RACK, Michael, D-69123 Heidelberg (DE); MÜLLER, Ruth, D-67159 Friedelsheim (DE); LORENZ, Gisela, D-67434 Hambach (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(86) Internationale Anmeldenummer: EP9604576
(87) Internationale Veröffentlichungsnummer: WO9716427

(56) Entgegenhaltungen:
- WO-A-95/18789
- WO-A-95/21153
- WO-A-95/21154
- PATENT ABSTRACTS OF JAPAN vol. 00, no. 0 & JP 08 127563 A (NIPPON NOHYAKU CO LTD), 21.Mai 1996,

## Beschreibung

Die vorliegende Erfindung betrifft Pyridylessigsäurederivate der Formel I 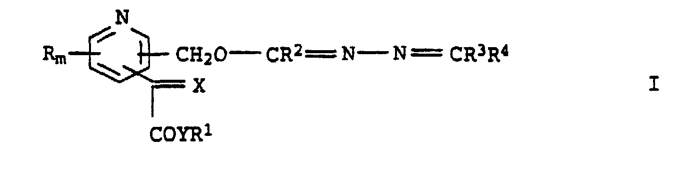 in der die Substituenten und der Index die folgende Bedeutung haben:
- X: NOCH₃, CHOCH₃ und CHCH₃;
- Y: Sauerstoff oder NR^{a};
R^{a} Wasserstoff oder C₁-C₄-Alkyl;
- R: Cyano, Nitro, Trifluormethyl, Halogen, C₁-C₄-Alkyl und C₁-C₄-Alkoxy;
- m: 0, 1 oder 2, wobei die Reste R verschieden sein können, wenn m für 2 steht;
- R¹: Wasserstoff oder C₁-C₄-Alkyl;
- R² und R³: unabhängig voneinander
Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Mercapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR^{b})-Aₙ-R^{c};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkoxy-C₁-C₆-alkyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Aryl-C₁-C₆-alkoxy, Hetaryl, Hetaryloxy, wobei die cyclischen Reste der sieben letztgenannten Substituenten partiell oder vollständig halogeniert sein können und/oder eine C₁-C₆-Alkylgruppe tragen können; C(=NOR^{b})-Aₙ-R^{c} oder NR^{f}-CO-D-R^{g};
   A Sauerstoff, Schwefel oder Stickstoff, wobei der Stickstoff Wasserstoff oder C₁-C₆-Alkyl trägt;
   D eine direkte Bindung, Sauerstoff oder NR^{h};
   n 0 oder 1;
   R^{b}, R^{c} unabhängig voneinander Wasserstoff oder C₁-C₆-Alkyl;
   R^{f} Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
   R^{g}, R^{h} unabhängig voneinander Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl;
- R⁴: eine der bei R² genannten Gruppen oder eine Gruppe CR^{d}=NOR^{e};
R^{d} eine der bei R² genannten Gruppen;
R^{e} Wasserstoff,
C₁-C₁₀-Alkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR^{b})-Aₙ-R^{c};
C₃-C₆-Cycloalkyl, Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{b})-Aₙ-R^{c} oder NR^{f}-CO-D-R^{g};
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Aus der Literatur sind Phenylessigsäurederivate zur Bekämpfung von tierischen Schädlingen und Schadpilzen bekannt (WO-A 95/18 789, WOP-A 95/21 153, WO-A 95/21 154, WO-A 95/21 156).

Der vorliegenden Erfindung lagen demgegenüber Verbindungen mit verbesserter Wirkung als Aufgabe zugrunde.

Demgemäß wurden die eingangs definierten Phenylessigsäurederivate I gefunden.

Außerdem wurden Verfahren und Zwischenprodukte zu ihrer Herstellung sowie sie enthaltende Mittel zur Bekämpfung tierischer Schädlinge und Schadpilze und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -C(X)-COYR¹ oder die Gruppierung -CH₂OCR²=N-N=CR³R⁴ aufgebaut wird.

Der Aufbau der Gruppierung -C(X)-COYR¹ ist beispielsweise aus der eingangs zitierten Literatur sowie aus EP-A 178 826, EP-A 370 629, EP-A 422 597, EP-A 460 575, EP-A 463 488, EP-A 472 300, EP-A 493 711, EP-A 534 216, EP-A 658 541, EP-A 658 542, EP-A 658 543, WO-A 90/07 493, WO-A 92/13 830 und WO-A 92/18 487 bekannt.

Die Art der Synthese der -CH₂OCR²=N-N=CR³R⁴ Seitenkette richtet sich im wesentlichen nach der Art des Substituenten R².
1. Für den Fall, daß R² nicht Halogen bedeutet, geht man beim Aufbau der Gruppierung CH₂OCR²=N-N=CR³R⁴ im allgemeinen so vor, daß man ein Benzylderivat der Formel II mit einem Carbonsäurehydrazid der Formel III umsetzt. 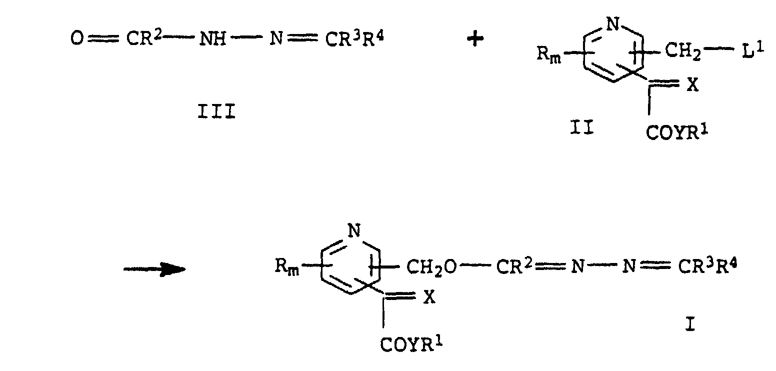
   L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370 f und Houben-Weyl, Bd. 10/1, S. 1189f beschriebenen Methoden.
   Das benötigte Carbonsäurehydrazid III erhält man beispielsweise nach literaturbekannten Methoden (*z.B. Maquestian, Tetrahedron 1987, 4185; Houben-Weyl, Methoden der organischen Chemie, Register der Stoffklassen Teil A, Bd. 16/2, S. 439-454*).
2. Verbindungen, in denen R² für ein Halogenatom steht, erhält man aus den entsprechenden Vorstufen, in denen der betreffende Rest für eine Hydroxygruppe steht, nach an sich bekannten Methoden (vgl. Houben-Weyl, Vol. E5, S. 631; J. Org. Chem. 36, 233 (1971); J. Org. Chem. 57, 3245 (1992)).
3. Verbindungen, in denen R² über ein O-, S- oder N-Atom an das Molekülgerüst gebunden ist, erhält man aus den entsprechenden Vorstufen, in denen der betreffende Rest für ein Halogenatom steht, nach an sich bekannten Methoden (vgl. Houben-Weyl, Bd. E5, S. 826 f und 1280 f, J. Org. Chem. 36, 233 (1971), J. Org. Chem. 46, 3623 (1981)).
4. Verbindungen, in denen R² über ein Sauerstoffatom an das Molekül gebunden ist, erhält man zum Teil auch aus den entsprechenden Vorstufen, in denen der betreffende Rest für eine Hydroxygruppe steht, nach an sich bekannten Methoden (vgl. Houben-Weyl, Bd. E 5, S. 826-829, Aust. J. Chem. 27, 1341-9 (1974)).
5. Nach einem bevorzugten Verfahren erhält man die Verbindungen I, in denen Y für Sauerstoff steht, indem man analog den in EP-A 493 711 und EP-A 534 216 beschriebenen Methoden den Aldehyd IV in das Cyanhydrin V überführt, V nach den Methoden der Pinner Reaktion zum Hydroxyester VI umsetzt, VI zum Ketoester VII oxidiert und VII entweder
   a) mit 0-Methylhydroxylamin oder einem O-Methylhydroxylammonium-salz,
   b) mit einem Ethylen-Wittig- oder -Wittig-Horner-Reagens, oder
   c) mit einem Methoxy-Wittig- oder -Wittig-Horner-Reagens in das Pyridin VIII überführt, VIII anschließend zum Halogenderivat IIa, b oder c (Hal = Cl, Br) umsetzt und IIa, b oder c mit einem Nucleophil III in Ia, b oder c überführt. 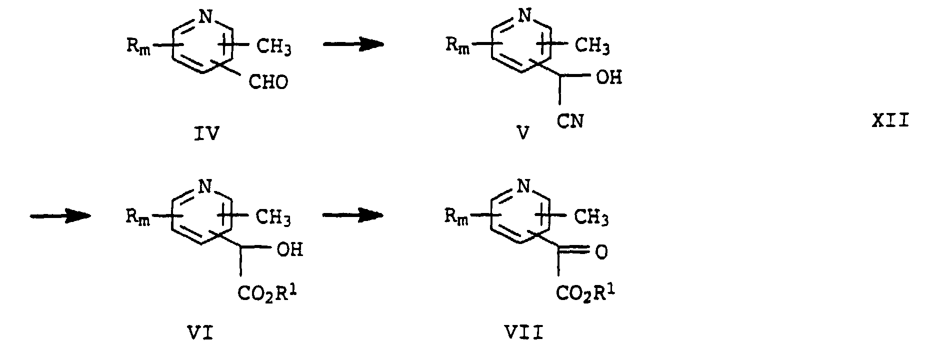 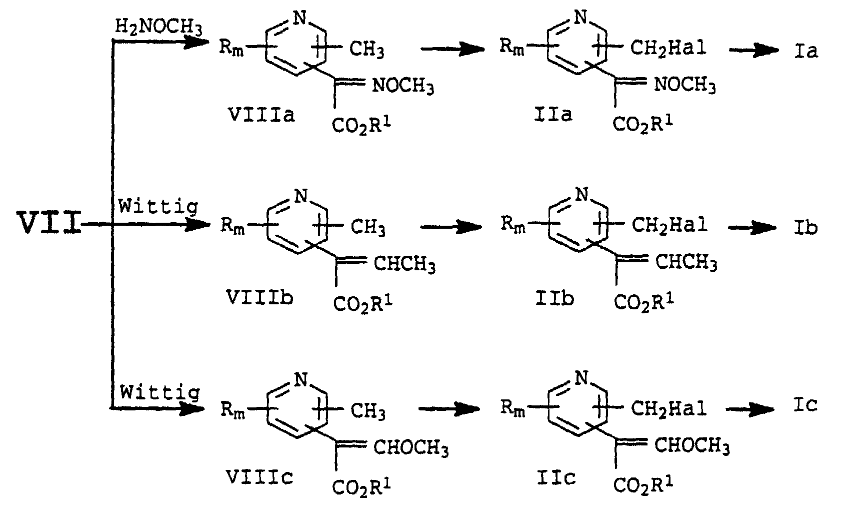
6. Nach einem weiteren bevorzugten Verfahren erhält man die Zwischenprodukte IIa, b oder c, indem man die unter Punkt 5. beschriebene Synthese ausgehend von geschützten Derivaten IX durchführt. 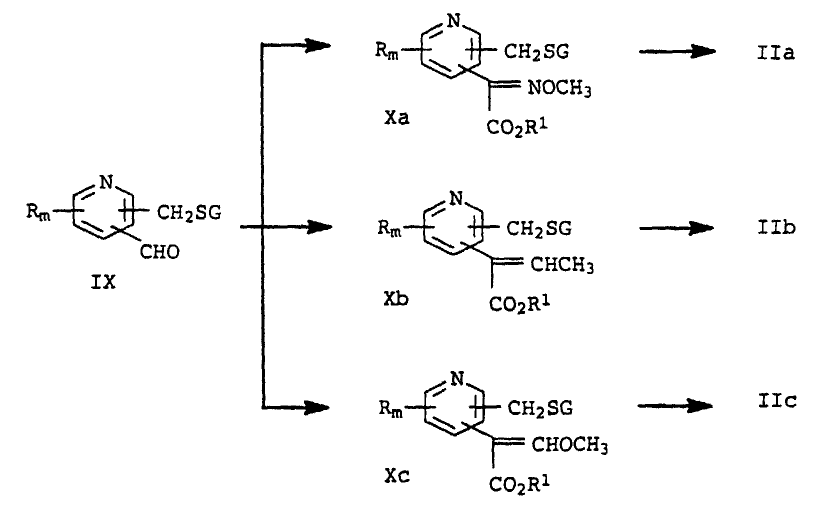
   In den Verbindungen IX und X steht SG für eine unter sauren Bedingungen abspaltbare Schutzgruppe wie Alkoxy, Aryloxy und Acyloxy. Die Abspaltung erfolgt nach den in der Literatur beschriebenen Methoden [*vgl*. *T.W. Greene, Protective Groups in Organic Synthesis, John Wiley & Sons 1981)* beispielsweise mit HBr, HCl oder BBr₃.
7. Verbindungen der Formel I, in denen Y für NR^{a} steht, erhält man aus den entsprechenden Verbindungen Ia in an sich bekannter Weise durch Umsetzung mit einem Amin der Formel VI. 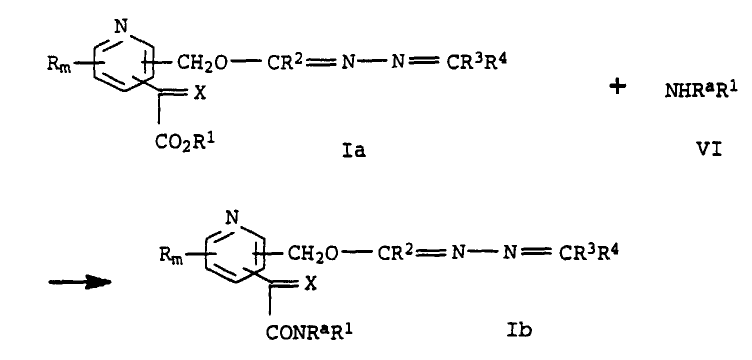
   Die Umsetzung des Esters Ia mit dem Amin erfolgt üblicherweise bei Temperaturen von 0°C bis 80°C, vorzugsweise von 15°C bis 60°C [ *vgl. EP-A 543 216*].
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Wasser, Methanol, Acetonitril und tert.-Butylmethylether. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, das Amin HNR^{a}R¹ in einem Überschuß bezogen auf Ia einzusetzen.

Die Verbindungen III sind bekannt oder können nach an sich bekannten Methoden hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=X-Doppelbindung werden hinsichtlich ihrer Wirksamkeit die E-Isomere der Verbindungen I bevorzugt (Konfiguration bezogen auf die OCH₃ bzw. CH₃-Gruppe im Verhältnis zur COYR¹-Gruppe).

In Bezug auf die -CR²=N-N=CR³R⁴ Doppelbindungen werden im allgemeinen hinsichtlich ihrer Wirksamkeit die *cis*-Isomere der Verbindungen I (Konfiguration bezogen auf den Rest R² im Verhältnis zur -N=CR³R⁴-Gruppe bzw. bezogen auf den Rest R³ im Verhältnis zur -N=CR²-Gruppe) bevorzugt.

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl**: geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methylpropyloxy, 2-Methylpropyloxy, 1,1-Dimethylethyloxy, Pentyloxy, 1-Methylbutyloxy, 2-Methylbutyloxy, 3-Methylbutyloxy, 2,2-Dimethylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und l-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-l-methyl-2-propenyl, 1-Ethyl-2-methyl-1-propenyl und 1-Ethyl-2-methyl-2-propenyl;
**Alkenyloxy**: geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino**: geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino**: monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder (Cycloalkenylamino) über ein Stickstoffatom an das Gerüst gebunden sind, z.B. Cyclobutenyl, Cyclopentenyl oder Cyclohexenyl.
**Cycloalkoxy bzw. Cycloalkylthio und Cycloalkylamino:** monocyclische Alkenylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 5-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,2,4-Triazolidin-5-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydrofur-4-yl, 2,3-Dihydrofur-5-yl, 2,5-Dihydrofur-2-yl, 2,5-Dihydrofur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,3-Dihydropyrazol-3-yl, 2,3-Dihydropyrazol-4-yl, 2,3-Dihydropyrazol-5-yl, 4,5-Dihydroisopyrazol-3-yl, 4,5-Dihydroisopyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl;
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino**: aromatische mono- oder polycyclische Kohlenwasserstoffreste, welche über ein Stickstoffatom an das Gerüst gebunden sind.
**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
   - 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl,5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,2,4-Triazol-3-yl, 1,3,4-Triazol-2-yl, 1,2,3,4-Tetrazolyl;
   - benzokondensiertes 5-gliedriges Heteroaryl, enthaltend ein bis drei Stirkstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin, bzw. die entsprechenden Oxy-, Thio-, Carbonyl- oder Sulfonylgruppen.
**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe *"partiell oder vollständig halogeniert*" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Hervorzuheben sind die Verbindungen der Formel I, in der die Substituenten und der Index die folgende Bedeutung haben:
- X: NOCH₃, CHOCH₃ und CHCH₃;
- Y: Sauerstoff oder NR^{a};
- R^{a}: Wasserstoff oder C₁-C₄-Alkyl;
- R: Cyano, Nitro, Trifluormethyl, Halogen, C₁-C₄-Alkyl und C₁-C₄-Alkoxy;
- m: 0, 1 oder 2, wobei die Reste R verschieden sein können, wenn m für 2 steht;
- R¹: Wasserstoff oder C₁-C₄-Alkyl;
- R² und R³: unabhängig voneinander
Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Mercapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR^{b})-Aₙ-R^{c};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{b})-Aₙ-R^{c} oder NR^{f}-CO-D-R^{g};
   A Sauerstoff, Schwefel oder Stickstoff, wobei der Stickstoff Wasserstoff oder C₁-C₆-Alkyl trägt;
   D eine direkte Bindung, Sauerstoff oder NR^{h};
   n 0 oder 1;
   R^{b}, R^{c} unabhängig voneinander Wasserstoff oder C₁-C₆-Alkyl;
   R^{f} Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
   R^{g}, R^{h} unabhängig voneinander Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl;
- R⁴: eine der bei R² genannten Gruppen oder eine Gruppe CR^{d}=NOR^{e};
R^{d} eine der bei R² genannten Gruppen;
R^{e} Wasserstoff,
C₁-C₁₀-Alkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die 12 letztgenannten Gruppen ihrerseits partiell oder vollständig halogeniert sein können oder eine bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR^{b})-An-R^{c};
C₃-C₆-Cycloalkyl, Aryl, Arylcarbonyl, Arylsulfonyl, Hetaryl, Hetarylcarbonyl oder Hetarylsulfonyl, wobei diese Reste partiell oder vollständig halogeniert sein können oder eine bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{b})-Aₙ-R^{c} oder NR^{f}-CO-D-R^{g};
sowie deren Salze.

Im Hinblick auf ihre biologische Wirkung sind Verbindungen der Formel I bevorzugt, in denen m für 0 oder 1, insbesondere 0, steht.

Für den Fall, daß m für 1 steht werden Verbindungen I bevorzugt, in denen R für Methyl, Trifluormethyl, Methoxy, Chlor und Fluor steht.

Besonders werden Verbindungen I bevorzugt, in denen X für NOCH₃ (Formel I.1) steht.

Außerdem werden Verbindungen I bevorzugt, in denen X für CHCH₃ (Formel I.2) steht.

Gleichermaßen werden Verbindungen I bevorzugt, in denen X für CHOCH₃ (Formel I.3) steht.

Daneben werden Verbindungen I besonders bevorzugt, in denen R¹ für Methyl steht.

Desweiteren werden Verbindungen I bevorzugt, in denen Y für Sauerstoff steht (Formel Ia).

Gleichermaßen werden Verbindungen I bevorzugt, in denen Y für NR^{a}, insbesondere NH, steht (Formel Ib).

Insbesondere werden auch Verbindungen I bevorzugt, in denen R² für Wasserstoff oder C₁-C₄-Alkyl steht.

Außerdem werden Verbindungen I besonders bevorzugt, in denen R² für Methyl steht.

Gleichermaßen besonders bevorzugt sind Verbindungen I, in denen R² für ggf. subst. Aryl oder Hetaryl steht.

Daneben werden Verbindungen I besonders bevorzugt, in denen R² für ggf. subst. Phenyl steht.

Desweiteren werden Verbindungen I besonders bevorzugt, in denen R² für Cyclopropyl steht.

Insbesondere werden auch Verbindungen I bevorzugt, in denen R³ für C₁-C₄-Alkyl, besonders Methyl, steht.

Außerdem werden Verbindungen I besonders bevorzugt, in denen R³ für Hetaryl, besonders ggf. subst. Pyridinyl, Isoxazolyl oder Pyrazolyl, steht.

Gleichermaßen besonders bevorzugt sind Verbindungen I, in denen R³ für Aryl, insbesondere ggf. subst. Phenyl, steht.

Daneben werden Verbindungen I besonders bevorzugt, in denen R³ für ggf. subst. Cycloalkyl, insbesondere Cyclopropyl, steht.

Insbesondere werden auch Verbindungen I bevorzugt, in denen R⁴ für C₁-C₄-Alkyl, besonders Methyl, steht.

Außerdem werden Verbindungen I besonders bevorzugt, in denen R⁴ für Aryl, besonders ggf. subst. Phenyl, steht.

Gleichermaßen besonders bevorzugt sind Verbindungen I, in denen R⁴ für Hetaryl, insbesondere ggf. subst. Pyridinyl, Isoxazolyl und Pyrazolyl, steht.

Daneben werden Verbindungen I besonders bevorzugt, in denen R⁴ für die Gruppe CR^{d}=NOR^{e} steht.

Daneben werden Verbindungen I bevorzugt, in denen der Rest CX-COYR¹ in 2-Position des Pyridylrings gebunden ist.

Desweiteren werden Verbindungen I bevorzugt, in denen der Rest CX-COYR¹ in 3-Position des Pyridylrings gebunden ist.

Außerdem werden Verbindungen I bevorzugt, in denen der Rest CX-COYR¹ in 4-Position des Pyridylrings gebunden ist.

Daneben werden Verbindungen I bevorzugt, in denen der Rest CH₂O-R²=N-N=CR³R⁴ in 2-Position des Pyridylrings gebunden ist.

Desweiteren werden Verbindungen I bevorzugt, in denen der Rest CH₂O-R²=N-N=CR³R⁴ in 3-Position des Pyridylrings gebunden ist.

Außerdem werden Verbindungen I bevorzugt, in denen der Rest CH₂O-R²=N-N=CR³R⁴ in 4-Position des Pyridylrings gebunden ist.

Insbesondere werden auch Verbindungen I bevorzugt, in denen R^{d} für C₁-C₄-Alkyl, besonders Methyl, steht.

Außerdem werden auch Verbindungen I bevorzugt, in denen R^{d} für Hetaryl, besonders ggf. subst. Pyridinyl, Isoxazolyl und Pyrazolyl, steht.

Daneben werden auch Verbindungen I bevorzugt, in denen R^{d} für Aryl, besonders ggf. subst. Phenyl, steht.

Gleichermaßen werden auch Verbindungen I bevorzugt, in denen R^{d} für ggf. subst. Cycloalkyl, besonders Cyclopropyl, steht.

Insbesondere werden auch Verbindungen I bevorzugt, in denen R^{e} für C₁-C₄-Alkyl, besonders Methyl, steht.

Außerdem werden auch Verbindungen I bevorzugt, in denen R^{e} für ggf. subst. Alkenyl, besonders Allyl und trans-Chlorallyl, steht.

Gleichermaßen werden auch Verbindungen I bevorzugt, in denen R^{e} für ggf. subst. Alkinyl, besonders Propargyl, Brom- und Iodpropargyl steht.

Desweiteren werden auch Verbindungen I bevorzugt, in denen R^{e} für Alkoxyalkyl, besonders C₁-C₂-Alkoxy-C₁-C₂-alkyl, steht.

Daneben werden auch Verbindungen I bevorzugt, in denen R^{e} für Arylalkyl, besonders im Phenylteil ggf. subst. Phenyl-C₁-C₂-alkyl, steht.

Gleichermaßen werden auch Verbindungen I bevorzugt, in denen R^{e} für ggf. subst. Hetaryl oder Hetarylalkyl steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der allgemeinen Formel IA.1, in denen R² Methyl bedeutet, R³ Methyl bedeutet und R⁴ für durch R^{x} substituiertes Phenyl steht wobei R^{x} für eine Verbindung jeweils einer Zeile der Tabelle A entspricht

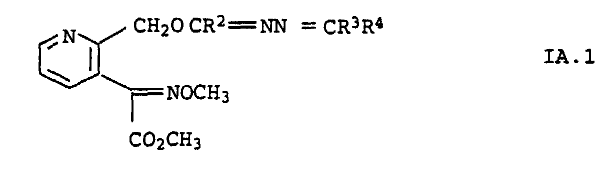

### Tabelle 2

Verbindungen der allgemeinen Formel IA.2, in denen R² Methyl bedeutet, R³ Methyl bedeutet und R⁴ für durch R^{x} substituiertes Phenyl steht wobei R^{x} für eine Verbindung jeweils einer Zeile der Tabelle A entspricht

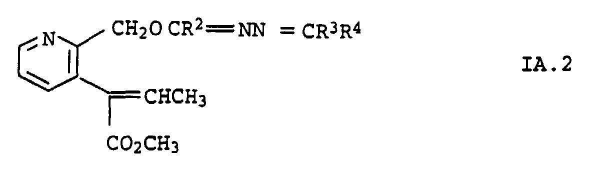

### Tabelle 3

Verbindungen der allgemeinen Formel IA.3, in denen R² Methyl bedeutet, R³ Methyl bedeutet und R⁴ für durch R^{x} substituiertes Phenyl steht wobei R^{x} für eine Verbindung jeweils einer Zeile der Tabelle A entspricht

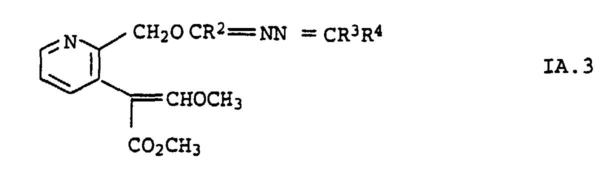

### Tabelle 4

Verbindungen der allgemeinen Formel IB.1, in denen R² Methyl bedeutet, R³ Methyl bedeutet und R⁴ für durch R^{x} substituiertes Phenyl steht wobei R^{x} für eine Verbindung jeweils einer Zeile der Tabelle A entspricht

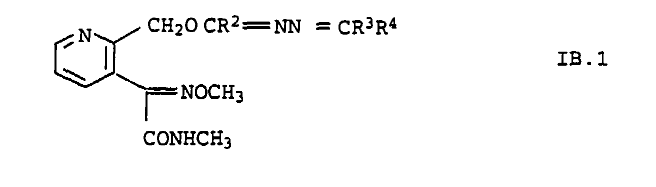

### Tabelle 5

Verbindungen der Formel IA.1, in denen die Kombination der Reste R², R³ und R⁴ für eine Verbindung jeweils einer Zeile der Tabelle B entspricht

### Tabelle 6

Verbindungen der Formel IA.2, in denen die Kombination der Reste R², R³ und R⁴ für eine Verbindung jeweils einer Zeile der Tabelle B entspricht

### Tabelle 7

Verbindungen der Formel IA.3, in denen die Kombination der Reste R², R³ und R⁴ für eine Verbindung jeweils einer Zeile der Tabelle B entspricht

### Tabelle 8

Verbindungen der Formel IB.1, in denen die Kombination der Reste R², R³ und R⁴ für eine Verbindung jeweils einer Zeile der Tabelle B entspricht

### Tabelle 9

Verbindungen der Formel IA.1.1, in denen R², R^{d} und R^{e} jeweils Methyl bedeuten und R³ für durch R^{x} substituiertes Phenyl steht, wobei R^{x} für eine Verbindung jeweils einer Zeile der Tabelle A entspricht

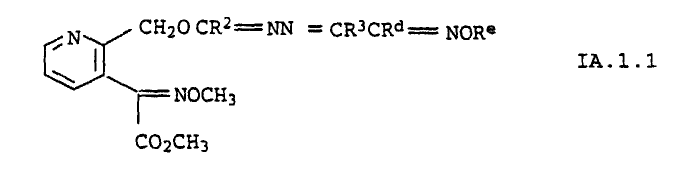

### Tabelle 10

Verbindungen der Formel IA.2.1, in denen R², R^{d} und R^{e} jeweils Methyl bedeuten und R³ für durch R^{x} substituiertes Phenyl steht, wobei R^{x} für eine Verbindung jeweils einer Zeile der Tabelle A entspricht

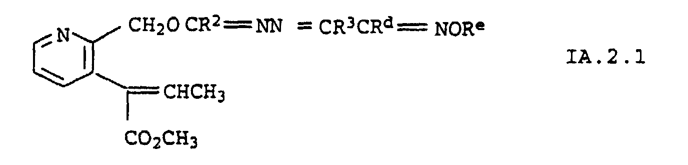

### Tabelle 11

Verbindungen der Formel IA.3.1, in denen R², R^{d} und R^{e} jeweils Methyl bedeuten und R³ für durch R^{x} substituiertes Phenyl steht, wobei R^{x} für eine Verbindung jeweils einer Zeile der Tabelle A entspricht

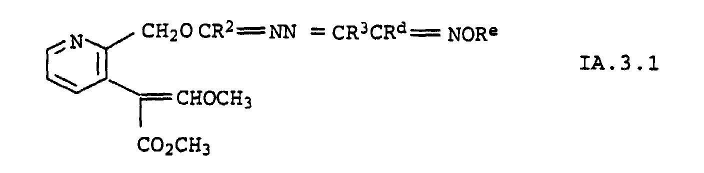

### Tabelle 12

Verbindungen der Formel IB.1.1, in denen R², R^{d} und R^{e} jeweils Methyl bedeuten und R³ für durch R^{x} substituiertes Phenyl steht, wobei R^{x} für eine Verbindung jeweils einer Zeile der Tabelle A entspricht

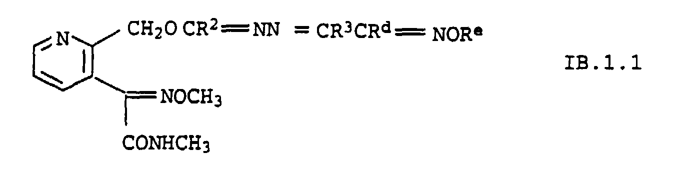

### Tabelle 13

Verbindungen der Formel IA.1.1, in denen R² für Ethyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 14

Verbindungen der Formel IA.2.1, in denen R² für Ethyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 15

Verbindungen der Formel IA.3.1, in denen R² für Ethyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 16

Verbindungen der Formel IB.1.1, in denen R² für Ethyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 17

Verbindungen der Formel IA.1.1, in denen R² für Cyclopropyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 18

Verbindungen der Formel IA.2.1, in denen R² für Cyclopropyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 19

Verbindungen der Formel IA.3.1, in denen R² für Cyclopropyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 20

Verbindungen der Formel IB.1.1, in denen R² für Cyclopropyl steht, R^{d} und R^{e} jeweils Methyl bedeuten und R³ für durch R^{x} substituiertes Phenyl steht, wobei R^{x} für eine Verbindung jeweils einer Zeile der Tabelle A entspricht

### Tabelle 21

Verbindungen der Formel IA.1.1, in denen R² für Methyl steht, R³ und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 22

Verbindungen der Formel IA.2.1, in denen R² für Methyl steht, R³ und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 23

Verbindungen der Formel IA.3.1, in denen R² für Methyl steht, R³ und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 24

Verbindungen der Formel IB.1.1, in denen R² für Methyl steht, R³ und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 25

Verbindungen der Formel IA.1.1, in denen R³ für Ethyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 26

Verbindungen der Formel IA.2.1, in denen R³ für Ethyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 27

Verbindungen der Formel IA.3.1, in denen R³ für Ethyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 28

Verbindungen der Formel IB.1.1, in denen R³ für Ethyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 29

Verbindungen der Formel IA.1.1, in denen R³ für iso-Propyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 30

Verbindungen der Formel IA.2.1, in denen R³ für iso-Propyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 31

Verbindungen der Formel IA.3.1, in denen R³ für iso-Propyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 32

Verbindungen der Formel IB.1.1, in denen R³ für iso-Propyl steht, R² und R^{e} jeweils Methyl bedeuten und R^{d} für durch R^{x} substituiertes Phenyl steht, wobei R^{x} für eine Verbindung jeweils einer Zeile der Tabelle A entspricht

### Tabelle 33

Verbindungen der Formel IA.1.1, in denen R^{e} für Ethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 34

Verbindungen der Formel IA.2.1, in denen R^{e} für Ethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 35

Verbindungen der Formel IA.3.1, in denen R^{e} für Ethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 36

Verbindungen der Formel IB.1.1, in denen R^{e} für Ethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 37

Verbindungen der Formel IA.1.1, in denen R^{e} für n-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 38

Verbindungen der Formel IA.2.1, in denen R^{e} für n-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 39

Verbindungen der Formel IA.3.1, in denen R^{e} für n-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 40

Verbindungen der Formel IB.1.1, in denen R^{e} für n-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 41

Verbindungen der Formel IA.1.1, in denen R^{e} für iso-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 42

Verbindungen der Formel IA.2.1, in denen R^{e} für iso-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 43

Verbindungen der Formel IA.3.1, in denen R^{e} für iso-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 44

Verbindungen der Formel IB.1.1, in denen R^{e} für iso-Propyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 45

Verbindungen der Formel IA.1.1, in denen R^{e} für tert.-Butyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 46

Verbindungen der Formel IA.2.1, in denen R^{e} für tert.-Butyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 47

Verbindungen der Formel IA.3.1, in denen R^{e} für tert.-Butyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 48

Verbindungen der Formel IB.1.1, in denen R^{e} für tert.-Butyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 49

Verbindungen der Formel IA.1.1, in denen R^{e} für Benzyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 50

Verbindungen der Formel IA.2.1, in denen R^{e} für Benzyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 51

Verbindungen der Formel IA.3.1, in denen R^{e} für Benzyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 52

Verbindungen der Formel IB.1.1, in denen R^{e} für Benzyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 53

Verbindungen der Formel IA.1.1, in denen R^{e} für Propargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 54

Verbindungen der Formel IA.2.1, in denen R^{e} für Propargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 55

Verbindungen der Formel IA.3.1, in denen R^{e} für Propargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 56

Verbindungen der Formel IB.1.1, in denen R^{e} für Propargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 57

Verbindungen der Formel IA.1.1, in denen R^{e} für Brompropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 58

Verbindungen der Formel IA.2.1, in denen R^{e} für Brompropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 59

Verbindungen der Formel IA.3.1, in denen R^{e} für Brompropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 60

Verbindungen der Formel IB.1.1, in denen R^{e} für Brompropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 61

Verbindungen der Formel IA.1.1, in denen R^{e} für Jodpropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 62

Verbindungen der Formel IA.2.1, in denen R^{e} für Jodpropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 63

Verbindungen der Formel IA.3.1, in denen R^{e} für Jodpropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 64

Verbindungen der Formel IB.1.1, in denen R^{e} für Jodpropargyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 65

Verbindungen der Formel IA.1.1, in denen R^{e} für Allyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 66

Verbindungen der Formel IA.2.1, in denen R^{e} für Allyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 67

Verbindungen der Formel IA.3.1, in denen R^{e} für Allyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 68

Verbindungen der Formel IB.1.1, in denen R^{e} für Allyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 69

Verbindungen der Formel IA.1.1, in denen R^{e} für trans-Chlorallyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 70

Verbindungen der Formel IA.2.1, in denen R^{e} für trans-Chlorallyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 71

Verbindungen der Formel IA.3.1, in denen R^{e} für trans-Chlorallyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 72

Verbindungen der Formel IB.1.1, in denen R^{e} für trans-Chlorallyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 73

Verbindungen der Formel IA.1.1, in denen R^{e} für Methoxyethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 74

Verbindungen der Formel IA.2.1, in denen R^{e} für Methoxyethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 75

Verbindungen der Formel IA.3.1, in denen R^{e} für Methoxyethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 76

Verbindungen der Formel IB.1.1, in denen R^{e} für Methoxyethyl steht, R² und R³ jeweils Methyl bedeuten und R^{d} für durch R^{x} substituiertes Phenyl steht, wobei R^{x} für eine Verbindung jeweils einer Zeile der Tabelle A entspricht

### Tabelle 77

Verbindungen der Formel IA.1.1, in denen R², R³, R^{d} und R^{e} die in Tabelle C genannte Bedeutung besitzen

### Tabelle 78

Verbindungen der Formel IA.2.1, in denen R², R³, R^{d} und R^{e} die in Tabelle C genannte Bedetung besitzen

### Tabelle 79

Verbindungen der Formel IA.3.1, in denen R², R³, R^{d} und R^{e} die in Tabelle C genannte Bedeutung besitzen

### Tabelle 80

Verbindungen der Formel IB.1.1, in denen R², R³, R^{d} und R^{e} die in Tabelle C genannte Bedeutung besitzen

**Tabelle A**

| Nr. | R^{x} |
|---|---|
| 01 | H |
| 02 | 2-F |
| 03 | 3-F |
| 04 | 4-F |
| 05 | 2,4-F₂ |
| 06 | 2,3-F₂ |
| 07 | 2,4,6-F₃ |
| 08 | 2,3,4,5,6-F₅ |
| 09 | 2-Cl |
| 10 | 3-Cl |
| 11 | 4-Cl |
| 12 | 2,3-Cl₂ |
| 13 | 2,4-Cl₂ |
| 14 | 2,5-Cl₂ |
| 15 | 2,6-Cl₂ |
| 16 | 3,4-Cl₂ |
| 17 | 3,5-Cl₂ |
| 18 | 2,3,4-Cl₃ |
| 19 | 2,3,5-Cl₃ |
| 20 | 2,3,6-Cl₃ |
| 21 | 2,4,5-Cl₃ |
| 22 | 2,4,6-Cl₃ |
| 23 | 3,4,5-Cl₃ |
| 24 | 2,3,4,6-Cl₄ |
| 25 | 2,3,5,6-Cl₄ |
| 26 | 2,3,4,5,6-Cl₅ |
| 27 | 2-Br |
| 28 | 3-Br |
| 29 | 4-Br |
| 30 | 2,4-Br₂ |
| 31 | 2,5-Br₂ |
| 32 | 2,6-Br₂ |
| 33 | 2,4,6-Br₃ |
| 34 | 2,3,4,5,6-Br₅ |
| 35 | 2-I |
| 36 | 3-I |
| 37 | 4-I |
| 38 | 2,4-I₂ |
| 39 | 2-Cl, 3-F |
| 40 | 2-Cl, 4-F |
| 41 | 2-Cl, 5-F |
| 42 | 2-Cl, 6-F |
| 43 | 2-Cl, 3-Br |
| 44 | 2-Cl, 4-Br |
| 45 | 2-Cl, 5-Br |
| 46 | 2-Cl, 6-Br |
| 47 | 2-Br, 3-Cl |
| 48 | 2-Br, 4-Cl |
| 49 | 2-Br, 5-Cl |
| 50 | 2-Br, 6-Cl |
| 51 | 2-Br, 3-F |
| 52 | 2-Br, 4-F |
| 53 | 2-Br, 5-F |
| 54 | 2-Br, 6-F |
| 55 | 2-F, 3-Cl |
| 56 | 2-F, 4-Cl |
| 57 | 2-F, 5-Cl |
| 58 | 4-F, 3-Cl |
| 59 | 5-F, 3-Cl |
| 60 | 4-Br, 3-Cl |
| 61 | 5-Br, 3-Cl |
| 62 | 3-F, 4-Cl |
| 63 | 3-F, 4-Br |
| 64 | 3-Br, 4-Cl |
| 65 | 4-F, 3-Br |
| 66 | 2,6-Cl₂, 4-Br |
| 67 | 2-CH₃ |
| 68 | 3-CH₃ |
| 69 | 4-CH₃ |
| 70 | 2,3-(CH₃)₂ |
| 71 | 2,4-(CH₃)₂ |
| 72 | 2,5-(CH₃)₂ |
| 73 | 2,6-(CH₃)₂ |
| 74 | 3,4-(CH₃)₂ |
| 75 | 3,5-(CH₃)₂ |
| 76 | 2,3,4-(CH₃)₃ |
| 77 | 2,3,5-(CH₃)₃ |
| 78 | 2,3,6-(CH₃)₃ |
| 79 | 2,4,5-(CH₃)₃ |
| 80 | 2,4,6-(CH₃)₃ |
| 81 | 3,4,5-(CH₃)₃ |
| 82 | 2,3,4,6-(CH₃)₄ |
| 83 | 2,3,5,6-(CH₃)₄ |
| 84 | 2,3,4,5,6-(CH₃)₅ |
| 85 | 2-C₂H₅ |
| 86 | 3-C₂H₅ |
| 87 | 4-C₂H₅ |
| 88 | 2,4-(C₂H₅)₂ |
| 89 | 2,6-(C₂H₅)₂ |
| 90 | 3,5-(C₂H₅)₂ |
| 91 | 2,4,6-(C₂H₅)₃ |
| 92 | 2-n-C₃H₇ |
| 93 | 3-n-C₃H₇ |
| 94 | 4-n-C₃H₇ |
| 95 | 2-i-C₃H₇ |
| 96 | 3-i-C₃H₇ |
| 97 | 4-i-C₃H₇ |
| 98 | 2,4-(i-C₃H₇)₂ |
| 99 | 2,6-(i-C₃H₇)₂ |
| 100 | 3,5-(i-C₃H₇)₂ |
| 101 | 2-s-C₄H₉ |
| 102 | 3-s-C₄H₉ |
| 103 | 4-s-C₄H₉ |
| 104 | 2-t-C₄H₉ |
| 105 | 3-t-C₄H₉ |
| 106 | 4-t-C₄H₉ |
| 107 | 4-n-C₉H₁₉ |
| 108 | 2-CH₃, 4-t-C₄H₉ |
| 109 | 2-CH₃, 6-t-C₄H₉ |
| 110 | 2-CH₃, 4-i-C₃H₇ |
| 111 | 2-CH₃, 5-i-C₃H₇ |
| 112 | 3-CH₃, 4-i-C₃H₇ |
| 113 | 2-c-C₆H₁₁ |
| 114 | 3-c-C₆H₁₁ |
| 115 | 4-c-C₆H₁₁ |
| 116 | 2-Cl, 4-C₆H₅ |
| 117 | 2-Br, 4-C₆H₅ |
| 118 | 2-OCH₃ |
| 119 | 3-OCH₃ |
| 120 | 4-OCH₃ |
| 121 | 2-OC₂H₅ |
| 122 | 3-OC₂H₅ |
| 123 | 4-OC₂H₅ |
| 124 | 2-O-n-C₃H₇ |
| 125 | 3-O-n-C₃H₇ |
| 126 | 4-O-n-C₃H₇ |
| 127 | 2-O-i-C₃H₇ |
| 128 | 3-O-i-C₃H₇ |
| 129 | 4-O-i-C₃H₇ |
| 130 | 2-O-n-C₆H₁₃ |
| 131 | 3-O-n-C₆H₁₃ |
| 132 | 4-O-n-C₆H₁₃ |
| 133 | 2-OCH₂C₆H₅ |
| 134 | 3-OCH₂C₆H₅ |
| 135 | 4-OCH₂C₆H₅ |
| 136 | 2-O(CH₂)₂C₆H₅ |
| 137 | 4-O(CH₂)₂C₆H₅ |
| 138 | 2,3-(OCH₃)₂ |
| 139 | 2,4-(OCH₃)₂ |
| 140 | 2,5-(OCH₃)₂ |
| 141 | 2,6-(OCH₃)₂ |
| 142 | 3,4-(OCH₃)₂ |
| 143 | 3,5-(OCH₃)₂ |
| 144 | 2-O-t-C₄H₉ |
| 145 | 3-O-t-C₄H₉ |
| 146 | 4-O-t-C₄H₉ |
| 147 | 3-(3'-Cl-C₆H₄) |
| 148 | 4-(4'-Cl-C₆H₄) |
| 149 | 2-OC₆H₅ |
| 150 | 3-OC₆H₅ |
| 151 | 4-OC₆H₅ |
| 152 | 2-O-(2'-F-C₆H₄) |
| 153 | 3-O-(3'-Cl-C₆H₄) |
| 154 | 4-O-(4'-CH₃-C₆H₄) |
| 155 | 2,3,6-(CH₃)₃, 4-F |
| 156 | 2,3,6-(CH₃)₃, 4-Cl |
| 157 | 2,3,6-(CH₃)₃, 4-Br |
| 158 | 2,4-(CH₃)₂, 6-F |
| 159 | 2,4-(CH₃)₂, 6-Cl |
| 160 | 2,4-(CH₃)₂, 6-Br |
| 161 | 2-i-C₃H₇, 4-Cl, 5-CH₃ |
| 162 | 2Cl, 4-NO₂ |
| 163 | 4-Cl, 2-NO₂ |
| 164 | 2-OCH₃, 4-NO₂ |
| 165 | 2,4-Cl₂, 5-NO₂ |
| 166 | 2,4-Cl₂, 6-NO₂ |
| 167 | 2,6-Cl₂, 4-NO₂ |
| 168 | 2,6-Br₂, 4-NO₂ |
| 169 | 2,6-I₂, 4-NO₂ |
| 170 | 2-CH₃, 4-Cl, 5-i-C₃H₇ |
| 171 | 2-CO₂CH₃ |
| 172 | 3-CO₂CH₃ |
| 173 | 4-CO₂CH₃ |
| 174 | 2-CH₂OCH₃ |
| 175 | 3-CH₂OCH₃ |
| 176 | 4-CH₂OCH₃ |
| 177 | 2-CH₃, 4-CO-i-C₃H₇ |
| 178 | 2-CH₃, 4-C(CH₃)=NOCH₃ |
| 179 | 2-CH₃, 4-C(CH₃)=NOC₂H₅ |
| 180 | 2-CH₃, 4-C(CH₃)=NO-n-C₃H₇ |
| 181 | 2-CH₃, 4-C(CH₃)=NO-i-PC₃H₇ |
| 182 | 2,5-(CH₃)₂, 4-C(CH₃)=NOCH₃ |
| 183 | 2,5-(CH₃)₂, 4-C(CH₃)=NOC₂H₅ |
| 184 | 2,5-(CH₃)₂, 4-C(CH₃)=NO-n-C₃H₇ |
| 185 | 2,5-(CH₃)₂, 4-C(CH₃)=NO-i-C₃H₇ |
| 186 | 2-C₆H₅ |
| 187 | 3-C₆H₅ |
| 188 | 4-C₆H₅ |
| 189 | 2-(2'-F-C₆H₄) |
| 190 | 2-CH₃, 5-Br |
| 191 | 2-CH₃, 6-Br |
| 192 | 3-CH₃, 2-Cl |
| 193 | 4-CH₃, 2-Cl |
| 194 | 5-CH₃, 2-Cl |
| 195 | 3-CH₃, 2-F |
| 196 | 4-CH₃, 2-F |
| 197 | 5-CH₃, 2-F |
| 198 | 3-CH₃, 2-Br |
| 199 | 4-CH₃, 2-Br |
| 200 | 5-CH₃, 2-Br |
| 201 | 3-CH₃, 4-Cl |
| 202 | 3-CH₃, 5-Cl |
| 203 | 3-CH₃, 4-F |
| 204 | 3-CH₃, 5-F |
| 205 | 3-CH₃, 4-Br |
| 206 | 3-CH₃, 5-Br |
| 207 | 4-CH₃, 3-F |
| 208 | 4-CH₃, 3-Cl |
| 209 | 4-CH₃, 3-Br |
| 210 | 4,5-(CH₃)₂, 2-Cl |
| 211 | 4,5-(CH₃)₂, 2-Br |
| 212 | 3,5-(CH₃)₂, 2-Cl |
| 213 | 3,5-(CH₃)₂, 2-Br |
| 214 | 2,6-Cl₂, 4-CH₄ |
| 215 | 2,6-F₂, 4-CH₄ |
| 216 | 2,6-Br₂, 4-CH₄ |
| 217 | 2,4-Br₂, 6-CH₄ |
| 218 | 2,4-F₂, 6-CH₄ |
| 219 | 2,4-Cl₂, 6-CH₄ |
| 220 | 2,6-(CH₃)₂, 4-F |
| 221 | 2,6-(CH₃)₂, 4-Cl |
| 222 | 2,6-(CH₃)₂, 4-Br |
| 223 | 3,5-(CH₃)₂, 4-F |
| 224 | 3,5-(CH₃)₂, 4-Cl |
| 225 | 3,5-(CH₃)₂, 4-Br |
| 226 | 2-CF₃ |
| 227 | 3-CF₃ |
| 228 | 4-CF₃ |
| 229 | 2-OCF₃ |
| 230 | 3-OCF₃ |
| 231 | 4-OCF₃ |
| 232 | 3-OCH₂CHF₂ |
| 233 | 2-NO₂ |
| 234 | 3-NO₂ |
| 235 | 4-NO₂ |
| 236 | 2-CN |
| 237 | 3-CN |
| 238 | 4-CN |
| 239 | 2-CH₃, 3-Cl |
| 240 | 2-CH₃, 4-Cl |
| 241 | 2-CH₃, 5-Cl |
| 242 | 2-CH₃, 6-Cl |
| 243 | 2-CH₃, 3-F |
| 244 | 2-CH₃, 4-F |
| 245 | 2-CH₃, 5-F |
| 246 | 2-CH₃, 6-F |
| 247 | 2-CH₃, 3-Br |
| 248 | 2-CH₃, 4-Br |
| 249 | 2-CH₃, 5-Br |
| 250 | 2-CH₃, 6-Br |
| 251 | 2,5-F₂ |
| 252 | 2,6-F₂ |
| 253 | 3,4-F₂ |
| 254 | 3,5-F₂ |
| n = neo; i = iso; s = sekundär; t = tertiär; c = cyclo | |

**Tabelle B**

| Nr. | R² | R³ | R⁴ |
|---|---|---|---|
| 1 | H | CH₃ | Phenyl |
| 2 | C₂H₅ | CH₃ | Phenyl |
| 3 | n-C₃H₇ | CH₃ | Phenyl |
| 4 | i-C₃H₇ | CH₃ | Phenyl |
| 5 | Cyclopropyl | CH₃ | Phenyl |
| 6 | Pyridyl-2 | CH₃ | Phenyl |
| 7 | Pyridyl-3 | CH₃ | Phenyl |
| 8 | Pyridyl-4 | CH₃ | Phenyl |
| 9 | 5-CH₃-Isoxazolyl-3 | CH₃ | Phenyl |
| 10 | Phenyl | CH₃ | Phenyl |
| 11 | CH₃ | H | Phenyl |
| 12 | CH₃ | C₂H₅ | Phenyl |
| 13 | CH₃ | n-C₃H₇ | Phenyl |
| 14 | CH₃ | i-C₃H₇ | Phenyl |
| 15 | CH₃ | Cyclopropyl | Phenyl |
| 16 | CH₃ | Pyridinyl-2 | Phenyl |
| 17 | CH₃ | Pyridinyl-3 | Phenyl |
| 18 | CH₃ | Pyridinyl-4 | Phenyl |
| 19 | CH₃ | 3-CH₃-Isoxazolyl-5 | Phenyl |
| 20 | CH₃ | Phenyl | Phenyl |
| 21 | H | CH₃ | CH₃ |
| 22 | CH₃ | CH₃ | CH₃ |
| 23 | C₂H₅ | CH₃ | CH₃ |
| 24 | n-C₃H₇ | CH₃ | CH₃ |
| 25 | i-C₃H₇ | CH₃ | CH₃ |
| 26 | Cyclopropyl | CH₃ | CH₃ |
| 27 | Pyridyl-2 | CH₃ | CH₃ |
| 28 | Pyridyl-3 | CH₃ | CH₃ |
| 29 | Pyridyl-4 | CH₃ | CH₃ |
| 30 | 5-CH₃-Isoxazolyl-3 | CH₃ | CH₃ |
| 31 | Phenyl | CH₃ | CH₃ |
| 32 | CH₃ | H | CH₃ |
| 33 | CH₃ | C₂H₅ | CH₃ |
| 34 | CH₃ | n-C₃H₇ | CH₃ |
| 35 | CH₃ | i-C₃H₇ | CH₃ |
| 36 | CH₃ | Cyclopropyl | CH₃ |
| 37 | CH₃ | Pyridinyl-2 | CH₃ |
| 38 | CH₃ | Pyridinyl-3 | CH₃ |
| 39 | CH₃ | Pyridinyl-4 | CH₃ |
| 40 | CH₃ | 3-CH₃-Isoxazolyl-5 | CH₃ |
| 41 | CH₃ | Phenyl | CH₃ |

**Tabelle C**

| Nr. | R² | R³ | R^{d} | R^{e} |
|---|---|---|---|---|
| 1 | H | CH₃ | CH₃ | CH₃ |
| 2 | CH₃ | CH₃ | CH₃ | CH₃ |
| 3 | C₂H₅ | CH₃ | CH₃ | CH₃ |
| 4 | n-C₃H₇ | CH₃ | CH₃ | CH₃ |
| 5 | i-C₃H₇ | CH₃ | CH₃ | CH₃ |
| 6 | Cyclopropyl | CH₃ | CH₃ | CH₃ |
| 7 | Pyridyl-2 | CH₃ | CH₃ | CH₃ |
| 8 | Pyridyl-3 | CH₃ | CH₃ | CH₃ |
| 9 | Pyridyl-4 | CH₃ | CH₃ | CH₃ |
| 10 | 5-CH₃-Isoxazolyl-3 | CH₃ | CH₃ | CH₃ |
| 11 | Phenyl | CH₃ | CH₃ | CH₃ |
| 12 | CH₃ | H | CH₃ | CH₃ |
| 13 | CH₃ | C₂H₅ | CH₃ | CH₃ |
| 14 | CH₃ | n-C₃H₇ | CH₃ | CH₃ |
| 15 | CH₃ | i-C₃H₇ | CH₃ | CH₃ |
| 16 | CH₃ | Cyclopropyl | CH₃ | CH₃ |
| 17 | CH₃ | Pyridinyl-2 | CH₃ | CH₃ |
| 18 | CH₃ | Pyridinyl-3 | CH₃ | CH₃ |
| 19 | CH₃ | Pyridinyl-4 | CH₃ | CH₃ |
| 20 | CH₃ | 3-CH₃-Isoxazolyl-5 | CH₃ | CH₃ |
| 21 | CH₃ | CH₃ | H | CH₃ |
| 22 | H | CH₃ | C₂H₅ | CH₃ |
| 23 | CH₃ | CH₃ | n-C₃H₇ | CH₃ |
| 24 | C₂H₅ | CH₃ | i-C₃H₇ | CH₃ |
| 25 | n-C₃H₇ | CH₃ | Cyclopropyl | CH₃ |
| 26 | i-C₃H₇ | CH₃ | Pyridinyl-2 | CH₃ |
| 27 | Cyclopropyl | CH₃ | Pyridinyl-3 | CH₃ |
| 28 | Pyridyl-2 | CH₃ | Pyridinyl-4 | CH₃ |
| 29 | Pyridyl-3 | CH₃ | 3-CH₃-Isoxazolyl-5 | CH₃ |
| 30 | CH₃ | CH₃ | CH₃ | C₂H₅ |
| 31 | CH₃ | CH₃ | CH₃ | n-C₃H₇ |
| 32 | CH₃ | CH₃ | CH₃ | i-C₃H₇ |
| 33 | CH₃ | CH₃ | CH₃ | t-C₄H₉ |
| 34 | CH₃ | CH₃ | CH₃ | Benzyl |
| 35 | CH₃ | CH₃ | CH₃ | Propargyl |
| 36 | CH₃ | CH₃ | CH₃ | Brompropragyl |
| 37 | CH₃ | CH₃ | CH₃ | Iodpropargyl |
| 38 | CH₃ | CH₃ | CH₃ | Alkyl |
| 39 | CH₃ | CH₃ | CH₃ | trans-Chlorallyl |
| 40 | CH₃ | CH₃ | CH₃ | CH₃-O-CH₂-CH₂ |

Ebenso insbesondere bevorzugt sind die in Tabelle D angeführten Verbindungen I.

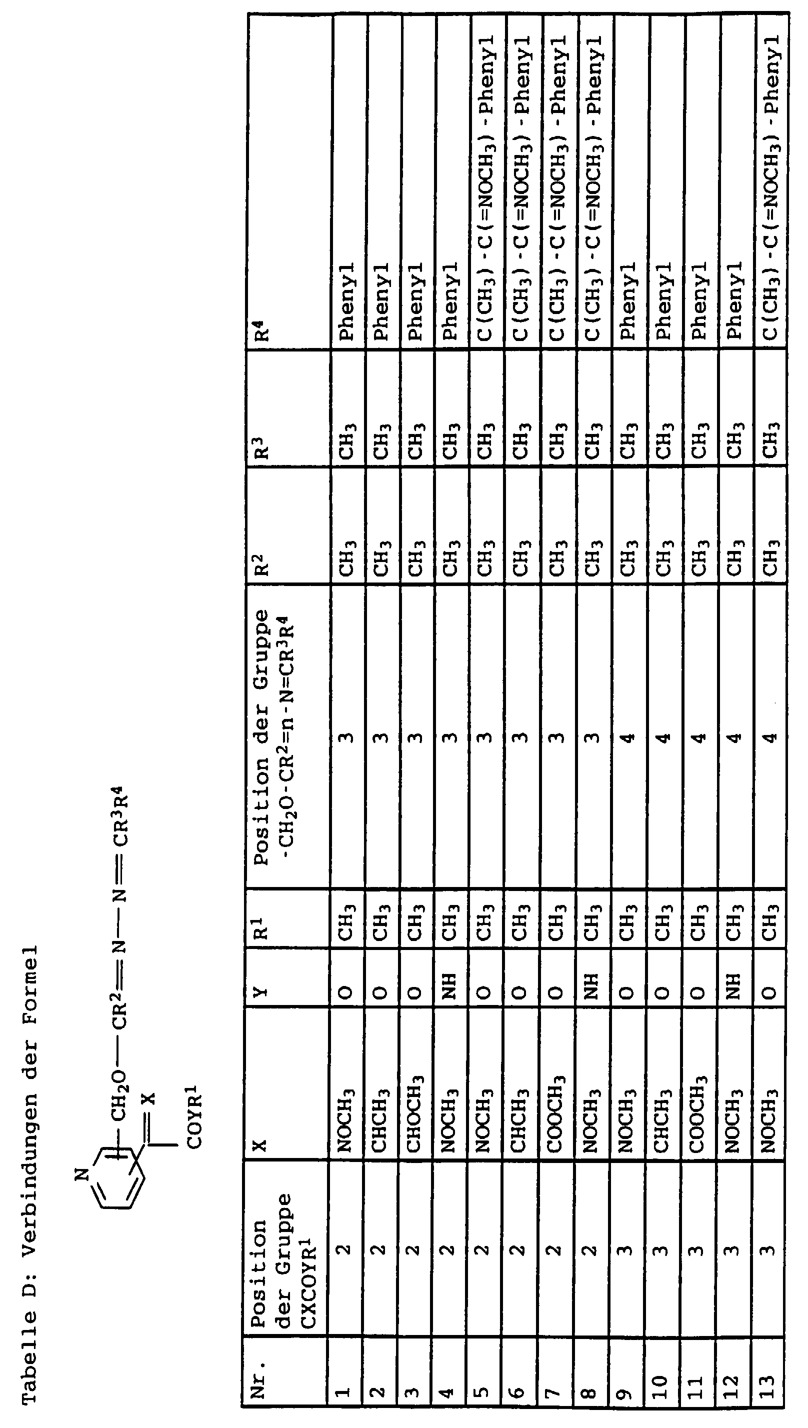

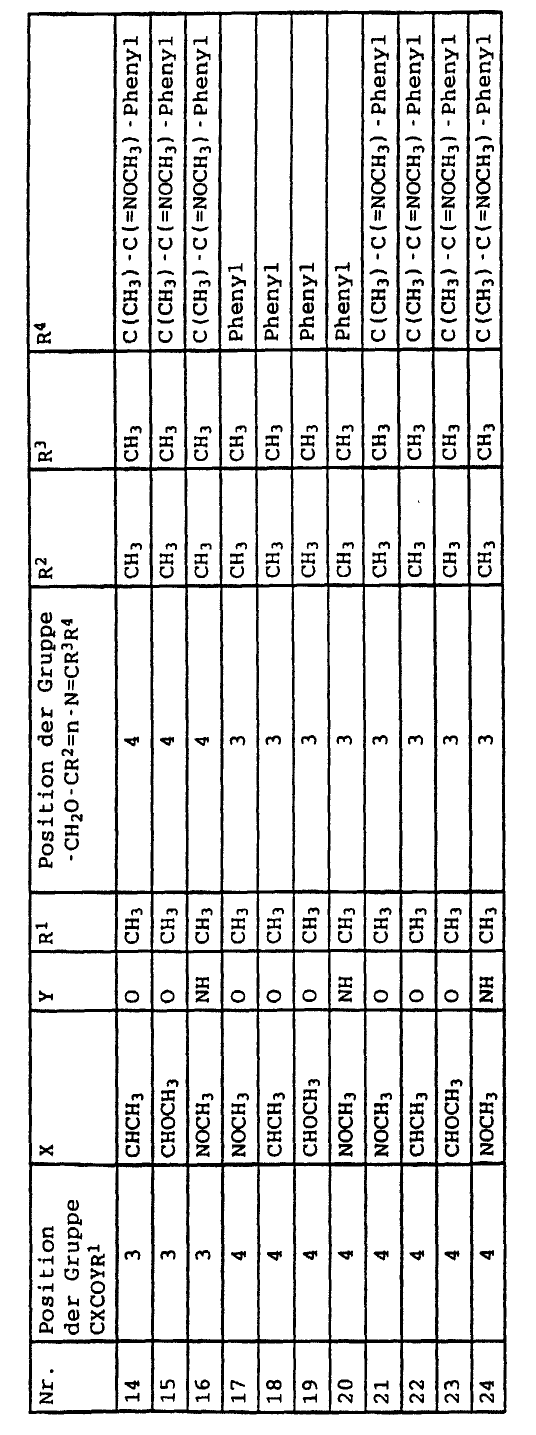

Ebenso betrifft die vorliegende Erfindung Zwischenprodukte der Formel II 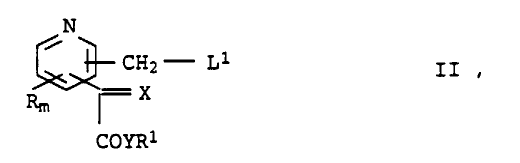 wobei R, R¹, X, Y und m die oben genannten Bedeutungen besitzen und L¹ eine nucleophil austauschbare Abgangsgruppe, wie Halogen z.B. Cl, Br, Sulfonat z.B. Mesylat, Triflat, Tosylat, C₁-C₆-Alkoxy wie oben genannt, Aryloxy wie oben genannt, C₁-C₆-Alkylcarbonyloxy wie oben genannt u.a. bedeuten kann. Bevorzugt kann L¹ Chlor, Brom, Mesylat, Triflat, Methoxy, Ethoxy oder Phenolat bedeuten. (Synthese vgl. S. 6ff dieser Anmeldung).

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindungen gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolinacetat, 2,4-Dichlor-6-(o-chloranilino)-s-triazin, 0,0-Diethylphthalimidophosphonothioat, 5-Amino-1-[bis-(dimethylamino)phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,4-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-[2,2,2-trichlor-1-(morpholin-4-yl)-ethyl-1]formamid, Piperazin-1,4-diylbis-(1-(2,2,2-trichlor-ethyl)formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze,
N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, l-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl) -N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl) -2-butanol, α-(2-Chlorphenyl)-α-(4-chlorphenyl)-5-pyrimidinmethanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3- (3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege stictiKalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius Kalifornicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus asKalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Kalotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Kalziumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100000 Gew.-Teilen Wasser erhält man eine wäßrige Dispersion, die 0,02 Gew.-% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Kalcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

1. Synthese von 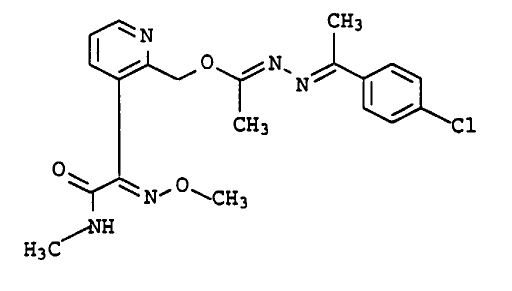
a) 2-Methyl-3-formylpyridin-cyanhydrin
   Eine Mischung von 33 g (0,5 Mol) KCN und 26 g (0,5 Mol) NH₄Cl in 200 ml Wasser und 30 g (0,24 Mol) 2-Methyl-3-formylpyridin (Chem. Pharm. Bull. 42 (1994), 1941; J. Med. Chem. 32 (1989), 583; J. Heterocycl. Chem. 28 (1991), 1315; J. Org. Chem. 43 (1978), 324) in 200 ml Diethylether wurden 2 Stunden bei Raumtemperatur gerührt, wobei das Produkt auskristallisierte. Der Festkörper wurde abgesaugt, mit Methyl-t-butylether gewaschen und im Stickstoffstrom getrocknet (Ausbeute: 19,3 g (54 %); Fp = 139°C).
   ¹H-NMR (DMSO-d₆; δ in ppm):
      8,5 (d, breit, 1H, Pyridyl); 7,9 (d, breit, 1H, Pyridyl); 7,3 (dd, 1H, Pyridyl); 7,2 (s, breit, 1H, OH); 5,9 (s, 1H, CH); 2,55 (s, 3H, CH3).
b) (2-Methylpyridyl-3)-α-hydroxy-essigsäuremethylester
   In eine Lösung von 22 g (0,15 Mol) 2-Methyl-3-formylpyridin-cyanhydrin (Beispiel 1a) in 250 ml Methanol wurden 15 g (0,4 Mol) gasförmige Salzsäure eingeleitet. Man rührte die Reaktionsmischung über Nacht bei Raumtemperatur und dampfte anschließend im Vakuum ein. Der Rückstand wurde in 200 ml Wasser aufgenommen und 1 Stunde zum Rückfluß erhitzt. Anschließend wurde die Reaktionsmischung auf Raumtemperatur abgekühlt, mit NaHCO₃-Lösung neutralisiert und mit Methylenchlorid extrahiert. Die vereinigten organischen Phasen wurden über MgSO₄ getrocknet und eingeengt. Der Rückstand wurde säulenchromatographisch mit Cyclohexan/Essigester-Gemischen gereinigt. Man erhält 3,0 g (11 %) der Titelverbindung als helles Öl.
   ¹H-NMR (CDCl₃; δ in ppm):
      8,35 (d, breit, 1H, Pyridyl); 7,7 (d, breit, 1H, Pyridyl); 7,15 (dd, 1H, Pyridyl); 5,4 (s, 1H, CH); 4,5 (s, sehr breit, 1H, OH); 3,75 (s, 3H, OCH₃); 2,6 (s, 3H, CH₃).
c) (2-Methylpyridyl-3)-glyoxylsäuremethylester
   Eine gerührte Mischung von 3 g (16 mmol) (2-Methylpyridyl-3)-α-hydroxy-essigsäuremethylester (Beispiel 1b) in 20 ml Methylenchlorid und 0,5 g (3,5 mmol) Na₂HPO₄, 0,6 g (5 mmol) NaH₂PO₄ und 0,2 g (1,6 mmol) KBr in 20 ml Wasser wurde mit einer Spatelspitze Tetramethylpyridin-N-oxyl und 10 ml 12,5 %iger Na-Hypochlorit-Lösung versetzt. Man rührte 1 Stunde bei Raumtemperatur, trennte anschließend die organische Phase ab und engte sie ein. Als Rückstand erhielt man 2,2 g (77 %) der Titelverbindung als gelbes Öl.
   ¹H-NMR (CDCl₃; δ in ppm):
      8,7 (d, breit, 1H, Pyridyl), 8,05 (d, breit, 1H, Pyridyl); 7,3 (dd, 1H, Pyridyl); 4,0 (s, 3H, OCH₃); 2,8 (s, 3H, CH3).
d) (2-Methylpyridyl-3)-glyoxylsäuremethylester-trans-O-methyloxim
   Eine Mischung von 2,2 g (12 mmol) (2-Methylpyridyl-3)-glyoxylsäuremethylester (Beispiel lc) und 1,5 g (18 mmol) o-Methylhydroxylamin-hydrochlorid in 20 ml Methanol wurde über Nacht bei Raumtemperatur gerührt. Anschließend wurde die Reaktionsmischung eingeengt. Der Rückstand wurde in Methylenchlorid aufgenommen und mit wenig Wasser extrahiert. Die organische Phase wurde über MgSO₄ getrocknet und eingeengt. Der Rückstand wurde säulenchromatographisch mit Cyclohexan-Essigester-Gemischen gereinigt. Man erhält 1,8 g (73 %) der Titelverbindung als helles Öl.
   ¹H-NMR (CDCl₃, δ in ppm):
      8,55 (d, breit, 1H, Pyridyl); 7,45 (d, breit, 1H, Pyridyl), 7,2 (dd, 1H, Pyridyl); 4,1 (s, 3H, OCH₃); 3,9 (s, 3H, OCH₃); 2,45 (s, 3H, CH3).
e) (2-Brommethylpyridyl-3)-glyoxylsäuremethylester-trans-O-methyloxim 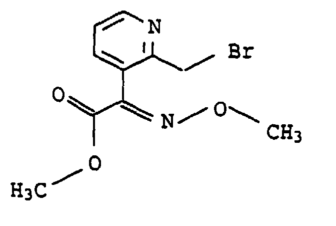
   Eine Mischung von 1,8 g (8,7 mmol) (2-Methylpyridyl-3)-glyoxylsäuremethylester-trans-O-methyloxim, 1,7 g (9,6 mmol) N-Bromsuccinimid und einer Spatelspitze Azoisobutyrodinitril in 30 ml CCl₄ wurde ca. 4 Stunden mit einer 300 W UV-Lampe bestrahlt, wobei sich die Reaktionsmischung auf Rückflußtemperatur erwärmte. Anschließend verdünnte man die Reaktionsmischung mit Methylenchlorid und extrahierte die organische Phase mit Wasser. Die organische Phase wurde mit Wasser extrahiert, über MgSO₄ getrocknet und eingeengt. Der Rückstand wurde säulenchromatographisch mit Cyclohexan/Essigester-Gemischen gereinigt. Man erhielt 1,1 g (44 %) der Titelverbindung als helles Öl.
   ¹H-NMR (CDCl₃; δ in ppm):
      8,65 (d, breit, 1H, Pyridyl); 7,55 (d, breit, 1H, Pyridyl); 7,3 (t, breit, 1H, Pyridyl); 4,4 (s, 2H, CH₂Br); 4,1 (s, 3H, OCH3); 3,9 (s, 3H, OCH₃).
f) N'-Acetyl-4-chloracetophenon-hydrazon 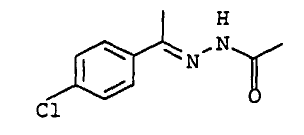
   Eine Mischung von 15,5 g (0,1 mol) 4-Chloracetophenon, 82 g (0,1 mol) N-Acetylhydrazid und einem Tropfen konz. Salzsäure in 100 ml Methanol wurde 3 Stunden auf 40-50°C erwärmt. Dabei kristallisierte das Produkt aus. Man kühlte die Reaktionsmischung ab und der auskristallisierte Festkörper wurde abfiltriert, mit Methanol/ Methyl-t-butylether gewaschen und trockengeblasen. Man erhielt 16 g (76 %) der Titelverbindung als farblosen Festkörper (Fp = 170-172°C).
   ¹H-NMR (CDCl₃; δ in ppm):
      9,75 (s, 1H, NH); 7,7 (d, 2H, Phenyl); 7,35 (d, 2H, Phenyl); 2,4 (s, 3H, CH3); 2,3 (s, 3H, CH₃).
g) Synthese von 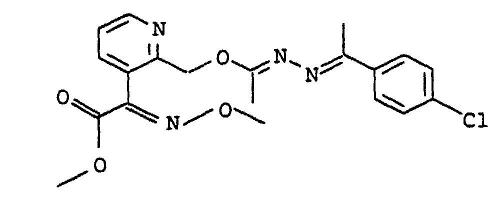
   Eine Mischung von 1,5 g (7 mmol) N'-Acetyl-4-chloracetophenon-hydrazon (Beispiel 1f) und 0,2 g (8 mmol) Natriumhydrid in 20 ml Dimethylformamid wurde 15 Minuten bei Raumtemperatur gerührt. Anschließend gab man 2 g (7 mmol) (2-Brommethylpyridyl-3)-glyoxylsäuremethylester-trans-O-methyloxim (Beispiel le) sowie 0,1 g Natriumiodid hinzu und rührte 2 Stunden bei Raumtemperatur. Dann verdünnte man die Reaktionsmischung mit Wasser und extrahierte die wäßrige Phase dreimal mit Methyl-t-butylether. Die vereinigten organischen Phasen wurden über MgSO₄ getrocknet und eingeengt. Der Rückstand wurde säulenchromatographisch mit Cyclohexan/Essigester-Gemischen gereinigt. Man erhielt 1,1 g (38 %) der Titelverbindung als hellgelbe Kristalle (Fp = 113-114°C).
   ¹H-NMR (CDCl₃; δ in ppm):
      8,65 (dd, 1H, Pyridyl); 7,75 (d, 2H, Phenyl); 7,6 (breit, 1H, Pyridyl); 7,35 (m, 3H, 2xPhenyl, lxPyridyl); 5,3 (s, 2H, OCH₂); 4,05 (s, 3H, OCH₃); 3,9 (s, 3H, OCH₃); 2,2 (s, 3H, CH₃); 2,1 (s, 3H, CH₃).
h) Synthese von 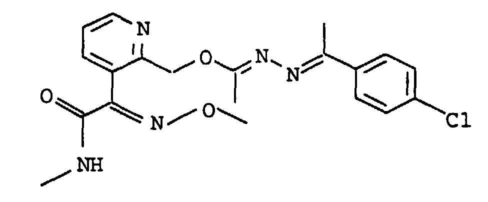
   Eine Mischung von 0,5 g (1,2 mmol) des Esters aus Beispiel 1g, 5 ml Tetrahydrofuran und 20 ml 40%iger Methylamin-Lösung würde 1 Stunde bei Raumtemperatur gerührt. Anschließend wurde die Reaktionsmischung im Vakuum eingeengt und der Rückstand wurde säulenchromatographisch mit Cyclohexan/Essigester-Gemischen gereinigt. Man erhielt 0,45 g (90 % der Titelverbindung als gelbes Öl.
   ¹H-NMR (CDCl₃; δ in ppm):
      8,65 (d, breit, 1H, Pyridyl); 7,75 (d, 2H, Phenyl); 7,6 (d, breit, 1H, Pyridyl); 7,35 (m, 3H, 2xPhenyl, lxPyridyl); 6,85 (s, sehr breit, 1H, NH); 5,3 (s, 2H, OCH₂); 3,95 (s, 3H, OCH₃); 2,9 (d, 3H, NCH₃); 2,2 (s, 3H, CH₃); 2,1 (s, 3H, CH₃).

In Analogie konnten die in Tabelle E aufgeführten Verbindungen dargestellt werden.

**Tabelle E:**

| Physikalische Daten einiger ausgewählter Verbindungen | | | | | | | |
|---|---|---|---|---|---|---|---|
| Nr. | Formel | R² | R³ | R⁴ | R^{d} | R^{e} | FP[°C] oder ¹H-NMR [ppm] |
| E.1 | IA.1 | CH₃ | CH₃ | 4-Cl-Phenyl | - | - | 113-114 |
| E.2 | IB.1 | CH₃ | CH₃ | 4-Cl-Phenyl | - | - | 3,95 (s, 3H); 2,9 (d, 3H) |
| E.3 | IA.1.1 CH₃ | | CH₃ | - | 4-Cl-Phenyl | CH₃ | 90-95 |
| E.4 | IB.1.1 | CH₃ | CH₃ | - | 4-Cl-Phenyl | CH₃ | 138-141 |

### Beispiele zur Wirkung gegen Schadpilze

Die fungizide Wirkung der Verbindungen der Formel I ließ sich durch folgende Versuche zeigen:

Die Wirkstoffe wurden als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirksamkeit gegen Weizenmehltau (Erysiphe graminis var. tritici)

Blätter von in Töpfen gewachsenen Weizenkeimlingen (Sorte "Frühgold") wurde mit einer Spritzbrühe, die 80 % Wirkstoff und 20 % Emulgiermittel in der Trockensubstanz enthielt, besprüht und 24 Stunden nach Behandlung mit Oidien (Sporen) von Weizenmehltau (Erysiphe graminis var. tritici) bestäubt. Die Versuchspflanzen wurden anschließend im Gewächshaus bei Temperaturen von 20 bis 22°C und bei einer relativen Luftfeuchte von 75 bis 80 % aufgestellt. Nach 7 Tagen wurde das Ausmaß der Mehltauentwicklung ermittelt.

| Verbindung | % Befall der Blätter nach Applikation von 250 ppm-haltiger Wirkstoffaufbereitung |
|---|---|
| E.2 | 25 |
| E.4 | 25 |
| Unbehandelt | 70 |

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Pyridylessigsäurederivate der Formel I 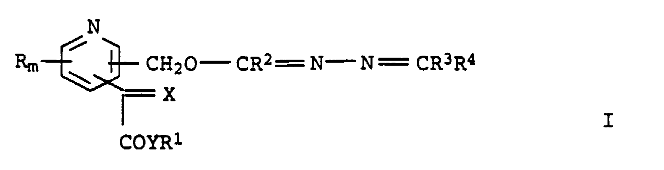 in der die Substituenten und der Index die folgende Bedeutung haben:
X NOCH₃, CHOCH₃ und CHCH₃;
Y Sauerstoff oder NR^{a};
R^{a} Wasserstoff oder C₁-C₄-Alkyl;
R Cyano, Nitro, Trifluormethyl, Halogen, C₁-C₄-Alkyl und C₁-C₄-Alkoxy;
m 0, 1 oder 2, wobei die Reste R verschieden sein können, wenn m für 2 steht;
R¹ Wasserstoff oder C₁-C₄-Alkyl;
R² und R³ unabhängig voneinander
Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Mercapto,
Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Ralogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR^{b})-Aₙ-R^{c};
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder eine bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Ralogenalkoxy, C₁-C₆-Alkoxycarbonyl, C₁-C₆-Alkylthio, C₁-C₆-Alkylamino, Di-C₁-C₆-alkylamino, C₁-C₆-Alkoxy-C₁-C₆-alkyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Aryl-C₁-C₆-alkoxy, Hetaryl, Hetaryloxy, wobei die cyclischen Reste der sieben letztgenannten Substituenten partiell oder vollständig halogeniert sein können und/oder eine C₁-C₆-Alkylgruppe tragen können; C(=NOR^{b})-Aₙ-R^{c} oder NR^{f}-CO-D-R^{g};
A Sauerstoff, Schwefel oder Stickstoff, wobei der Stickstoff Wasserstoff oder C₁-C₆-Alkyl trägt;
D eine direkte Bindung, Sauerstoff oder NR^{h};
n 0 oder 1;
R^{b}, R^{c} unabhängig voneinander Wasserstoff oder C₁-C₆-Alkyl;
R^{f} Wasserstoff, Hydroxy, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkoxy-C₁-C₆-alkoxy und C₁-C₆-Alkoxycarbonyl;
R^{g}, R^{h} unabhängig voneinander Wasserstoff, C₁-C₆-Alkyl, C₂-C₆-Alkenyl, C₂-C₆-Alkinyl, C₃-C₆-Cycloalkyl, C₃-C₆-Cycloalkenyl, Aryl, Aryl-C₁-C₆-alkyl, Hetaryl und Hetaryl-C₁-C₆-alkyl;
R⁴ eine der bei R² genannten Gruppen oder eine Gruppe CR^{d}=NOR^{e};
R^{d} eine der bei R² genannten Gruppen;
R^{e} Wasserstoff,
C₁-C₁₀-Alkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die zwölf letztgenannten Gruppen ihrerseits partiell oder vollständig halogeniert sein können oder eine bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C (=NOR^{b})-Aₙ-R^{c};
C₃-C₆-Cycloalkyl, Aryl, Arylcarbonyl, Arylsulfonyl, Hetaryl, Hetarylcarbonyl oder Hetarylsulfonyl, wobei diese Reste partiell oder vollständig halogeniert sein können oder eine bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy, C(=NOR^{b})-Aₙ-R^{c} oder NR^{f}-CO-D-R^{g};
sowie deren Salze.

2. Verbindungen der Formel I gemäß Anspruch 1, in denen X NOCH₃ und Y NR^{a} bedeutet.

3. Verbindungen der Formel I gemäß Anspruch 1, in denen m für 0 steht.

4. Verbindungen der Formel I gemäß Anspruch 1, in denen R¹ für Methyl steht.

5. Verfahren zur Herstellung der Verbindungen I gemäß Anspruch 1, in denen R² nicht Halogen bedeutet, dadurch gekennzeichnet, daß man ein Benzylderivat der Formel II 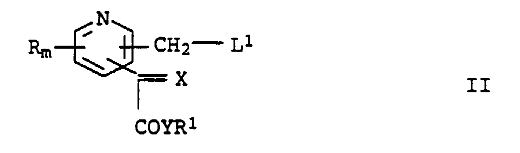 in der L¹ für eine nucleophil austauschbare Abgangsgruppe steht, in an sich bekannter Weise mit einem Carbonsäurehydrazid der Formel III
O=CR²-NH-N=CR³R⁴ III
umsetzt.

6. Verfahren zur Herstellung der Verbindungen I gemäß Anspruch 1, in denen Y für NR^{a} steht, dadurch gekennzeichnet, daß man den entsprechenden Phenylessigsäureester der Formel Ia 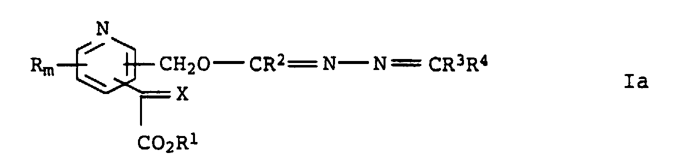 in an sich bekannter Weise mit einem Amin der Formel VI
NHR^{a}R¹ VI
umsetzt.

7. Zwischenprodukte der Formel II gemäß Anspruch 5.

8. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

9. Verwendung der Verbindungen I gemäß Anspruch 1 zur Herstellung eines zur Bekämpfung von Schädlingen oder Schadpilzen geeigneten Mittels.

10. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

11. Verfahren zur Bekämpfung von Schädlingen, dadurch gekennzeichnet, daß man die Schädlinge oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. A pyridylacetic acid derivative of the formula I 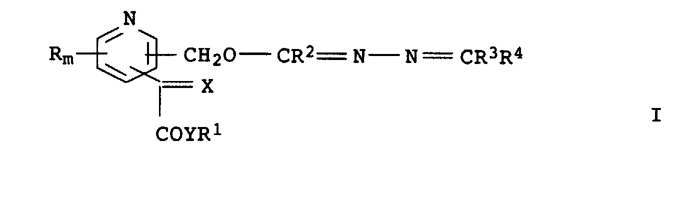 where the substituents and the index have the following meanings:
X is NOCH₃, CHOCH₃ and CHCH₃;
Y is oxygen or NR^{a};
R^{a} is hydrogen or C₁-C₄-alkyl;
R is cyano, nitro, trifluoromethyl, halogen, C₁-C₄-alkyl and C₁-C₄-alkoxy;
m is 0, 1 or 2, it being possible for the radicals R to be different when m is 2;
R¹ is hydrogen or C₁-C₄-alkyl;
R² and R³ independently of one another are
hydrogen, cyano, nitro, hydroxyl, amino, halogen, C₁-C₆-alkyl, C₁-C₆-alkoxy, C₁-C₆-alkylthio, C₁-C₆-alkylamino, di-C₁-C₆-alkylamino, C₂-C₆-alkenyl, C₂-C₆-alkenyloxy, C₂-C₆-alkenylthio, C₂-C₆-alkenylamino, N-C₂-C₆-alkenyl-N-C₁-C₆-alkylamino, C₂-C₆-alkynyl, C₂-C₆-alkynyloxy, C₂-C₆-alkynylthio, C₂-C₆-alkynylamino, N-C₂-C₆-alkynyl-N-C₁-C₆-alkylamino, it being possible for the hydrocarbon radicals of these groups to be partially or fully halogenated or to have attached to them one to three of the following radicals: cyano, nitro, hydroxyl, mercapto,
amino, carboxyl, aminocarbonyl, aminothiocarbonyl, halogen, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio, hetaryl-C₁-C₄-alkylthio, it being possible for the cyclic radicals, in turn, to be partially or fully halogenated and/or to have attached to them one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C(=NOR^{b})-Aₙ-R^{c};
C₃-C₆-cycloalkyl, C₃-C₆-cycloalkyloxy, C₃-C₆-cycloalkylthio, C₃-C₆-cycloalkylamino, N-C₃-C₆-cycloalkyl-N-C₁-C₆-alkylamino, C₃-C₆-cycloalkenyl, C₃-C₆-cycloalkenyloxy, C₃-C₆-cycloalkenylthio, C₃-C₆-cycloalkenylamino, N-C₃-C₆-cycloalkenyl-N-C₁-C₆-alkylamino, heterocyclyl, heterocyclyloxy, heterocyclylthio, heterocyclylamino, N-heterocyclyl-N-C₁-C₆-alkylamino, aryl, aryloxy, arylthio, arylamino, N-aryl-N-C₁-C₆-alkylamino, hetaryl, hetaryloxy, hetarylthio, hetarylamino, N-hetaryl-N-C₁-C₆-alkylamino, it being possible for the cyclic radicals to be partially or fully halogenated or to have attached to them one to three of the following groups: cyano, nitro, hydroxy, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkoxy-C₁-C₆-alkyl, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, aryl-C₁-C₆-alkoxy, hetaryl, hetaryloxy, it being possible for the cyclic radicals of the seven last-mentioned substituents to be partially or fully halogenated and/or to have attached to them a C₁-C₆-alkyl group; C(=NOR^{b})-Aₙ-R^{c} or NR^{f}-CO-D-R^{g};
A is oxygen, sulfur or nitrogen, the nitrogen having attached to it hydrogen or C₁-C₆-alkyl;
D is a direct bond, oxygen or NR^{h};
n is 0 or 1;
R^{b}, R^{c} independently of one another are hydrogen or C₁-C₆-alkyl;
R^{f} is hydrogen, hydroxyl, C₁-C₆-alkyl, C₂-C₆-alkenyl, C₂-C₆-alkynyl, C₁-C₆-alkoxy, C₂-C₆-alkenyloxy, C₂-C₆-alkynyloxy, C₁-C₆-alkoxy-C₁-C₆-alkyl, C₁-C₆-alkoxy-C₁-C₆-alkoxy and C₁-C₆-alkoxycarbonyl;
R^{g}, R^{h} independently of one another are hydrogen, C₁-C₆-alkyl, C₂-C₆-alkenyl, C₂-C₆-alkynyl, C₃-C₆-cycloalkyl, C₃-C₆-cycloalkenyl, aryl, aryl-C₁-C₆-alkyl, hetaryl and hetaryl-C₁-C₆-alkyl;
R⁴ is one of the groups mentioned under R² or a group CR^{d}=NOR^{e};
R^{d} is one of the groups mentioned under R²;
R^{e} is hydrogen,
C₁-C₁₀-alkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these radicals to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkyloxy, heterocyclyl, heterocyclyloxy, benzyl, benzyloxy, aryl, aryloxy, arylthio, hetaryl, hetaryloxy and hetarylthio, it being possible for the twelve last-mentioned groups, in turn, to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR^{b})-Aₙ-R^{c};
C₃-C₆-cycloalkyl, aryl, arylcarbonyl, arylsulfonyl, hetaryl, hetarylcarbonyl or hetarylsulfonyl, it being possible for these radicals to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy, C(=NOR^{b})-Aₙ-R^{c} or NR^{f}-CO-D-R^{g};
or a salt thereof.

2. A compound of the formula I as claimed in claim 1 where X is NOCH₃ and Y is NR^{a}.

3. A compound of the formula I as claimed in claim 1 where m is 0.

4. A compound of the formula I as claimed in claim 1 where R¹ is methyl.

5. A process for the preparation of the compounds I as claimed in claim 1 where R² is not halogen, which comprises reacting a benzyl derivative of the formula II 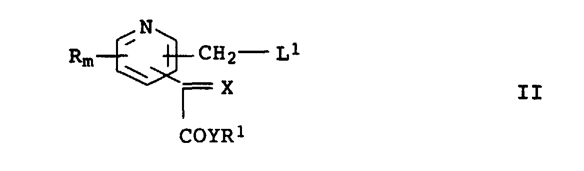 where L¹ is a nucleophilically exchangeable leaving group with a carbohydrazide of the formula III
O=CR²-NH-N=CR³R⁴ III
in a manner known per se.

6. A process for the preparation of the compounds I as claimed in claim 1 where Y is NR^{a}, which comprises reacting the corresponding phenylacetic ester of the formula Ia 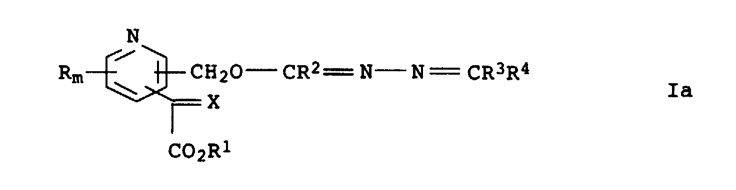 with an amine of the formula VI
NHR^{a}R¹ VI
in a manner known per se.

7. An intermediate of the formula II as claimed in claim 5.

8. A composition suitable for controlling pests or harmful fungi, comprising a solid or liquid carrier and a compound of the general formula I as claimed in claim 1.

9. The use of a compound I as claimed in claim 1 for the preparation of a composition suitable for controlling pests or harmful fungi.

10. A method of controlling harmful fungi, which comprises treating the fungi, or the materials, plants, the soil or seed to be protected against fungal infection, with an effective amount of a compound of the general formula I as claimed in claim 1.

11. Method of controlling pests, which comprises treating the pests, or the materials, plants, the soil or seed to be protected against them, with an effective amount of a compound of the general formula I as claimed in claim 1.

## Revendications

1. Dérivés d'acide pyridylacétique de formule I 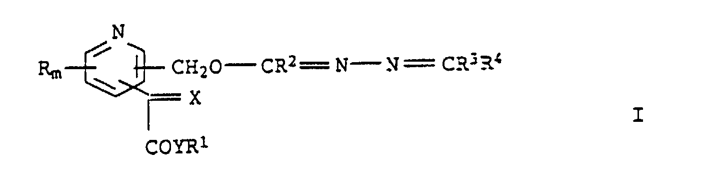 dans laquelle les substituants et les indices ont la signification suivante:
X NOCH₃, CHOCH₃ et CHCH₃,
Y oxygène ou NR^{a},
R^{a} hydrogène ou alkyle en C₁-C₄,
R cyano, nitro, trifluorométhyle, halogéno, alkyle en C₁-C₄ et alcoxy en C₁-C₄,
m 0, 1 ou 2, tandis que les restes R peuvent être différents lorsque m vaut 2,
R¹ hydrogène ou alkyle en C₁-C₄,
R² et R³, indépendamment l'un de l'autre,
hydrogène, cyano, nitro, hydroxy, amino, halogéno,
alkyle en C₁-C₆, alcoxy en C₁-C₆, alkylthio en C₁-C₆, alkylamino en C₁-C₆, di-alkyl(C₁-C₆)amino, alcényle en C₂-C₆, alcényloxy enC₂-C₆, alcénylthio en C₂-C₆, alcénylamino en C₂-C₆, N-alcényl(C₂-C₆)-N-alkylamino en C₁-C₆, tandis que les restes hydrocarbonés de ces groupes peuvent être partiellement ou totalement halogénés ou peuvent porter un à trois des restes suivants: cyano, nitro, hydroxy, mercapto,
amino, carbonyle, aminocarbonyle, aminothiocarbonyle, halogéno, alkyl(C₁-C₆)aminocarbonyle, di-alkyl(C₁-C₆)aminocarbonyle, alkyl(C₁-C₆)aminothiocarbonyle, di-alkyl(C₁-C₆)aminothiocarbonyle, alkyl (C₁-C₆) sulfonyle, alkyl (C₁-C₆) sulfoxyle, alcoxy en C₁-C₆, halogénoalcoxy en C₁-C₆, alcoxy(C₁-C₆)carbonyle, alkylthio en C₁-C₆, alkylamino en C₁-C₆, di-alkyl(C₁-C₆)amino, alcényloxy en C₂-C₆, cycloalkyle en C₃-C₆, cycloalkyloxy en C₃-C₆, hétérocyclyle, hétérocyclyloxy, aryle, aryloxy, aryl-alcoxy (C₁-C₄), arylthio, arylalkylthio(C₁-C₄), hétaryle, hétaryloxy, hétarylalcoxy(C₁-C₄), hétarylthio, hétarylalkylthio(C₁-C₄), tandis que les restes cycliques peuvent à leur tour être partiellement ou totalement halogénés et/ou peuvent porter un à trois des groupes suivants: cyano, nitro, hydroxy, mercapto, amino, carboxyle, aminocarbonyle, aminothiocarbonyle, alkyle en C₁-C₆, halogénoalkyle en C₁-C₆, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)aminocarbonyle, dialkyl(C₁-C₆)aminocarbonyle, alkyl(C₁-C₆)aminothiocarbonyle, di-alkyl(C₁-C₆)aminothiocarbonyle, alcényle en C₂-C₆, alcényloxy en C₂-C₆, benzyle, benzyloxy, aryle, aryloxy, arylthio, hétaryle, hétaryloxy, hétarylthio et C(=NOR^{b})-Aₙ-R^{c};
cycloalkyle en C₃-C₆, cycloalkyloxy en C₃-C₆, cycloalkylthio en C₃-C₆, cycloalkylamino en C₃-C₆, N-cycloalkyl(C₃-C₆)-N-alkyl(C₁-C₆)amino, cycloalcényle en C₃-C₆, cycloalcényloxy en C₃-C₆, cycloalcénylthio en C₃-C₆, cycloalcénylamino en C₃-C₆, N-cycloalcényl(C₃-C₆)-N-alkyl(C₁-C₆)amino, hétérocyclyle, hétérocyclyloxy, hétérocyclylthio, hétérocyclylamino, N-hétérocyclyl-N-alkyl(C₁-C₆)amino, aryle, aryloxy, arylthio, arylamino, N-aryl-N-alkyl(C₁-C₆)amino, hétaryle, hétaryloxy, hétarylthio, hétarylamino, N-hétaryl-N-alkyl(C₁-C₆)amino, tandis que les restes cycliques peuvent être partiellement ou totalement halogénés ou peuvent porter un à trois des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alcoxy(C₁-C₆)-alkyle(C₁-C₆), alkyl(C₁-C₆)aminocarbonyle, di-alkyl(C₁-C₆)aminocarbonyle, alkyl(C₁-C₆)aminothiocarbonyle, dialkyl(C₁-C₆)aminothiocarbonyle, alcényle en C₂-C₆, alcényloxy en C₂-C₆, benzyle, benzyloxy, aryle, aryloxy, aryl-alcoxy(C₁-C₆), hétaryle, hétaryloxy, tandis que les restes cycliques des sept substituants mentionnés en dernier peuvent être partiellement ou totalement halogénés et/ou peuvent porter un groupe alkyle en C₁-C₆; C(=NOR^{b})-Aₙ-R^{c} ou NR^{f}-CO-D-R^{g};
A oxygène, soufre ou azote, tandis que l'azote porte de l'hydrogène ou un alkyle en C₁-C₆;
D une liaison directe, de l'oxygène ou NR^{h};
n 0 ou 1;
R^{b}, R^{c}, indépendamment l'un de l'autre, hydrogène ou alkyle en C₁-C₆;
R^{f} hydrogène, hydroxy, alkyle en C₁-C₆, alcényle en C₂-C₆, alcynyle en C₂-C₆, alcoxy en C₁-C₆, alcényloxy en C₂-C₆, alcynyoxy en C₂-C₆, alcoxy(C₁-C₆)-alkyle(C₁-C₆), alcoxy(C₁-C₆)-alcoxy(C₁-C₆) et alcoxy(C₁-C₆)carbonyle;
R^{g}, R^{h}, indépendamment l'un de l'autre, hydrogène, alkyle en C₁-C₆, alcényle en C₂-C₆, alcynyle en C₂-C₆, cycloalkyle en C₃-C₆, cycloalcényle en C₃-C₆, aryle, aryl-alkyle(C₁-C₆), hétaryle et hétaryl-alkyle(C₁-C₆);
R⁴ un des groupes désignés par R² ou un groupe CR^{d}=NOR^{e};
R^{d} un des groupes désignés par R²;
R^{e} hydrogène;
alkyle en C₁-C₁₀, alcényle en C₂-C₁₀, alcynyle en C₂-C₁₀, alkyl(C₁-C₁₀)carbonyle, alcényl(C₂-C₁₀)carbonyle, alcynyl(C₃-C₁₀)carbonyle ou alkylsulfonyle en C₁-C₁₀, tandis que ces restes peuvent être partiellement ou totalement halogénés ou peuvent porter un à trois des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkylsulfonyle en C₁-C₆, alkylsulfoxyle en C₁-C₆, alcoxy en C₁-C₆, halogénoalcoxy en C₁-C₆, alcoxy(C₁-C₆)carbonyle, alkylthio en C₁-C₆, alkylamino en C₁-C₆, di-alkyl(C₁-C₆)amino, alkyl(C₁-C₆)aminocarbonyle, dialkyl(C₁-C₆)aminocarbonyle, alkyl(C₁-C₆)aminothiocarbonyle, di-alkyl(C₁-C₆)aminothiocarbonyle, alcényle en C₂-C₆, alcényloxy en C₂-C₆, cycloalkyle en C₃-C₆, cycloalkyloxy en C₃-C₆, hétérocyclyle, hétérocyclyloxy, benzyle, benzyloxy, aryle, aryloxy, arylthio, hétaryle, hétaryloxy et hétarylthio, tandis que les douze groupes mentionnés en dernier peuvent à leur tour être partiellement ou totalement halogénés ou peuvent porter un à trois des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkylsulfonyle en C₁-C₆, alkylsulfoxyle en C₁-C₆, cycloalkyle en C₃-C₆, alcoxy en C₁-C₆, halogénoalcoxy en C₁-C₆, alkyl(C₁-C₆)oxycarbonyle, alkylthio en C₁-C₆, alkylamino en C₁-C₆, di-alkyl(C₁-C₆)amino, alkyl(C₁-C₆)aminocarbonyle, di-alkyl(C₁-C₆) aminocarbonyle, alkyl(C₁-C₆)aminothiocarbonyle, dialkyl(C₁-C₆)aminothiocarbonyle, alcényle en C₂-C₆, alcényloxy en C₂-C₆, benzyl, benzyloxy, aryle, aryloxy, arylthio, hétaryle, hétaryloxy, hétarylthio ou C(=NOR^{b})-Aₙ-R^{c};
cycloalkyle en C₃-C₆, aryle, arylcarbonyle, arylsulfonyle, hétaryle, hétarylcarbonyle ou hétarylsulfonyle, tandis que ces restes peuvent être partiellement ou totalement halogénés ou peuvent porter un à trois des groupes suivants: cyano, nitro, hydroxy, mercapto, amino, carboxyle, aminocarbonyle, aminothiocarbonyle, halogéno, alkyle en C₁-C₆, halogénoalkyle en C₁-C₆, alkyl(C₁-C₆)carbonyle, alkylsulfonyle en C₁-C₆, alkylsulfoxyle en C₁-C₆, cycloalkyle en C₃-C₆, alcoxy en C₁-C₆, halogénoalcoxy en C₁-C₆, alkyl(C₁-C₆)oxycarbonyle, alkylthio en C₁-C₆, alkylamino en C₁-C₆, di-alkyl(C₁-C₆)amino, alkyl(C₁-C₆)aminocarbonyle, di-alkyl(C₁-C₆)aminocarbonyle, alkyl(C₁-C₆)aminothiocarbonyle, di-alkyl(C₁-C₆)aminothiocarbonyle, alcényle en C₂-C₆, alcényloxy en C₂-C₆, benzyle, benzyloxy, aryle, aryloxy, hétaryle, hétaryloxy, C(=NOR^{b})-Aₙ-R^{c} ou NR^{f}-CO-D-R^{g};
ainsi que leurs sels.

2. Composés de formule I selon la revendication 1, dans lesquels X représente NOCH₃ et Y NR^{a}.

3. Composés de formule I selon la revendication 1, dans lesquels m vaut 0.

4. Composés de formule I selon la revendication 1, dans lesquels R¹ désigne un méthyle.

5. Procédé pour la préparation de composés I selon la revendication 1, dans lesquels R² ne représente pas un halogène, caractérisé par le fait qu'on fait réagir un dérivé benzylé de formule II 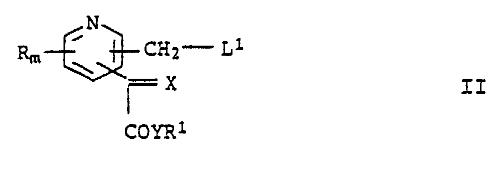 dans laquelle L¹ désigne un groupe éliminable échangeable par voie nucléophile, de manière connue en soi avec un hydrazide d'acide carboxylique de formule III
O=CR²-NH-N=CR³R⁴ III

6. Procédé pour la préparation des composés I selon la revendication 1, dans lesquels Y désigne NR^{a}, caractérisé par le fait qu'on fait réagir l'ester d'acide phénylacétique correspondant de formule Ia 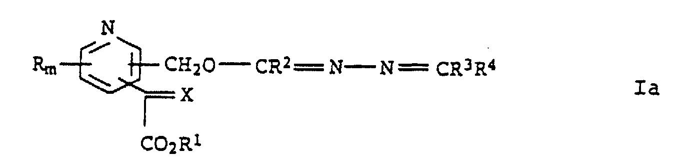 de manière connue en soi avec une amine de formule VI
NHR^{a}R¹ VI

7. Produits intermédiaires de formule II selon la revendication 5.

8. Moyen approprié pour la lutte contre les parasites ou les champignons nuisibles, contenant un support solide ou liquide et un composé de formule générale I selon la revendication 1.

9. Utilisation des composés I selon la revendication 1 pour la préparation d'un moyen approprié pour la lutte contre les parasites ou les champignons nuisibles.

10. Procédé pour lutter contre les champignons nuisibles, caractérisé par le fait qu'on traite les champignons ou les matières, plantes, sols ou semences à protéger contre une invasion de champignons avec une quantité efficace d'un composé de formule générale I selon la revendication 1.

11. Procédé pour lutter contre les parasites, caractérisé par le fait qu'on traite les parasites ou les matières, plantes, sols ou semences à protéger contre eux avec une quantité efficace d'un composé de formule générale I selon la revendication 1.
